(19) Europäisches Patentamt
European Patent Office
Office européen des brevets

(11) **EP 2 594 940 A1**

(12) **EUROPEAN PATENT APPLICATION**

(43) Date of publication:
**22.05.2013 Bulletin 2013/21**

(51) Int Cl.:
***G01N 33/543*** (2006.01)

(21) Application number: **11191453.7**

(22) Date of filing: **01.12.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.11.2011 US 560450 P**

(71) Applicant: **Koninklijke Philips Electronics N.V.
5621 BA Eindhoven (NL)**

(72) Inventor: **The designation of the inventor has not yet been filed**

(74) Representative: **Jungen, Carmen
Philips Intellectual Property &
Standards GmbH
Weißhausstraße 2
52066 Aachen (DE)**

(54) **Particle repulsion to enhance surface contact in magnetic particle immunoassays**

(57) The present invention relates to a device for detecting a target molecule within a sample comprising a sample container for the measurement of the target molecule within a sample, a magnetic particle, wherein said particle is functionalized with a first binding molecule capable of specifically binding to said target molecule, wherein said first binding molecule is attached to the particle, and a repulsive surface structure, which is directly attached to the surface of said particle, wherein said repulsive surface structure covers the surface of the magnetic particle so as to result in a specific net charge and/or steric repulsion of the magnetic particle, and a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface.

FIGURE 9

## Description

FIELD OF THE INVENTION

The present invention relates to a device for detecting a target molecule within a sample comprising a sample container for the measurement of the target molecule within a sample, a magnetic particle, wherein said particle is functionalized with a first binding molecule capable of specifically binding to said target molecule, wherein said first binding molecule is attached to the particle, and a repulsive surface structure, which is directly attached to the surface of said particle, wherein said repulsive surface structure covers the surface of the magnetic particle so as to result in a specific net charge and/or steric repulsion of the magnetic particle, and a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface. In a second aspect the invention describes a device for detecting a target molecule within a sample comprising a mixture of at least two types of magnetic particles, wherein one type is functionalized with a first binding molecule capable of specifically binding to said target molecule, wherein said first binding molecule is attached to the particle, and a second type is functionalized with a repulsive surface structure, which is directly attached to the surface of said particle, wherein said repulsive surface structure covers the surface of the magnetic particle so as to result in a specific net charge and/or steric repulsion of the magnetic particle, and a sensor surface comprising a second binding molecule, wherein said magnetic particles are capable of binding said second binding molecule of the sensor surface directly or indirectly. Furthermore, the invention describes a method of detecting the presence or amount of a target molecule within a sample as well as the use of a magnetic particle for detecting a target molecule within a sample.

BACKGROUND OF THE INVENTION

The demand for pervasive and effective healthcare moves the world of in vitro diagnostics towards integrated random-access and point-of care solutions. The achievement of such solutions is demanding: the tests needs to be rapid, sensitive, quantitative and accurate. Moreover, the platform on which the test is performed need to be easy to use and compact.

Affinity assays make use of biological molecules to capture specific target molecules from a sample and allow a determination of their concentration. Typically, affinity capture is achieved by dispersing nano- or microparticles coated with capture molecules into sample fluid (Luchini et al., 2008, Nano Lett., 8(1), 350 - 361). Typical affinity-based assays are therefore used in a huge number of applications such as diagnostic assays, detection of biomolecules in research such as proteins, peptides and nucleic acids thereby making use of affinity molecules such as, e.g. antibodies, which are typically characterized by a high binding affinity towards a specific biomolecule. In principle, the functionalized magnetic particles are attracted to a sensor surface, where the particles can indirectly, i.e. by virtue of a captured analyte or directly bind to capture probes such as antibodies printed on the surface. The number of bound particles is directly or inversely related to the amount of target molecules present I the sample. Typically, in such biosensor applications, the particles can be detected using any technique sensitive to a particle close at the surface; often such techniques are based on optical detection such as the detection of scattered light or frustrated total internal reflection (FTIR) as described for instance in Bruls et al., Lab Chip, 2009, 9. 2504-3510.

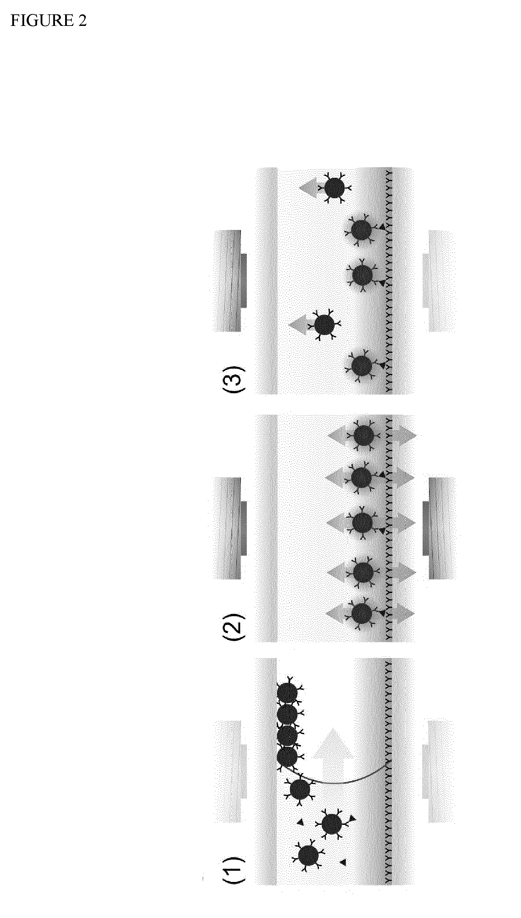

An important drawback of such affinity-assays as described in the prior art, however, is the fact that the binding of the functionalized particles having bound a target molecule to the surface is still very slow, rate-limiting and inefficient. One reason for this slow reaction can be the limited interaction of the particles with the surface. The inventors of the present application have observed that in such assays, the particles typically can diffuse away from the surface, when the magnetic field is switched off as can be seen in Fig. 2, thus decreasing the amount of time the particles spend near the surface in order to perform binding. Also the use of more particles in order to generate a top-layer of the particle cloud that is capable of pushing the particles at the bottom further down toward the surface has the disadvantage that binding chance per particle is lowered.

There is thus a strong need to provide means and methods to increase the particle-surface contact without increasing the amount of particles.

OBJECTS AND SUMMARY OF THE INVENTION

The present invention addresses these needs and provides means for enhancing the binding efficiency of particles to surfaces. The above objective is in particular accomplished by a device for detecting a target molecule within a sample comprising a sample container for the measurement of the target molecule within a sample, a magnetic particle, wherein said particle is functionalized with a first binding molecule capable of specifically binding to said target molecule, wherein said first binding molecule is attached to the particle, and a repulsive surface structure, which is directly attached to the surface of said particle, wherein said repulsive surface structure covers the surface of the magnetic particle so as to result in a specific net charge and/or steric repulsion of the magnetic particle, and a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface.

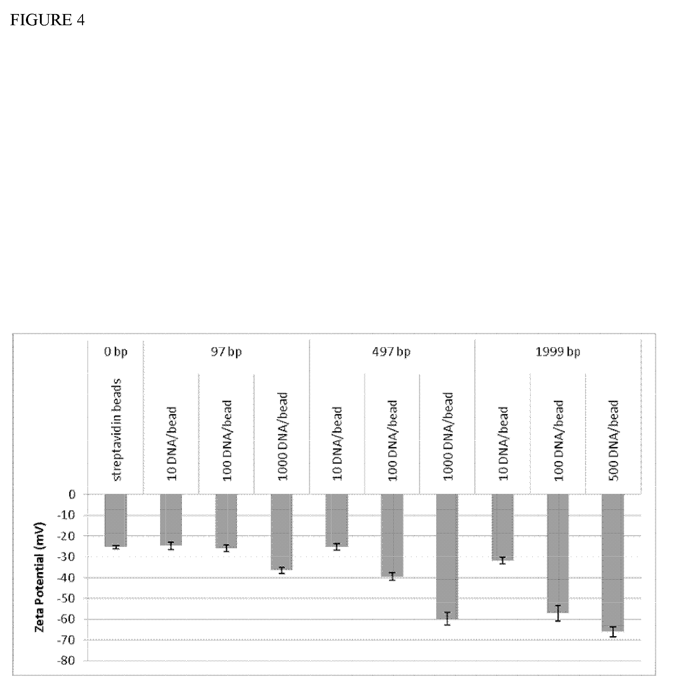

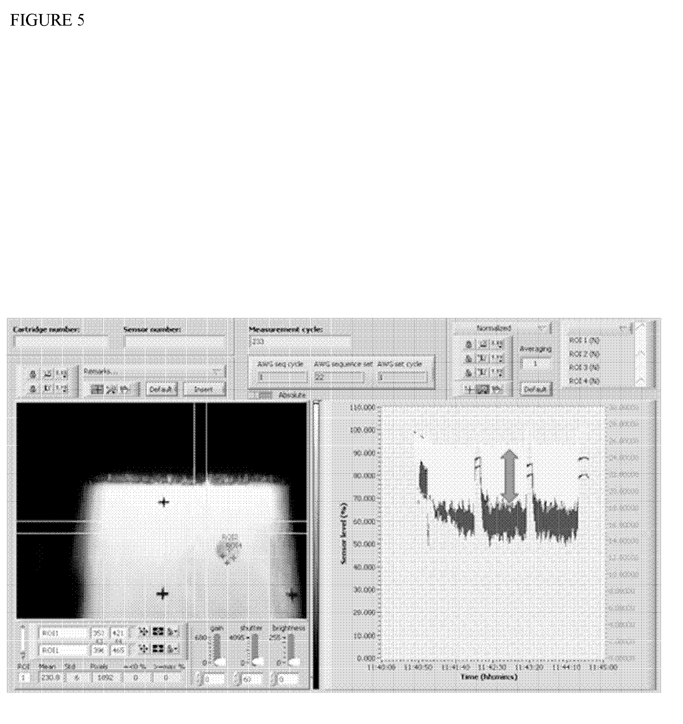

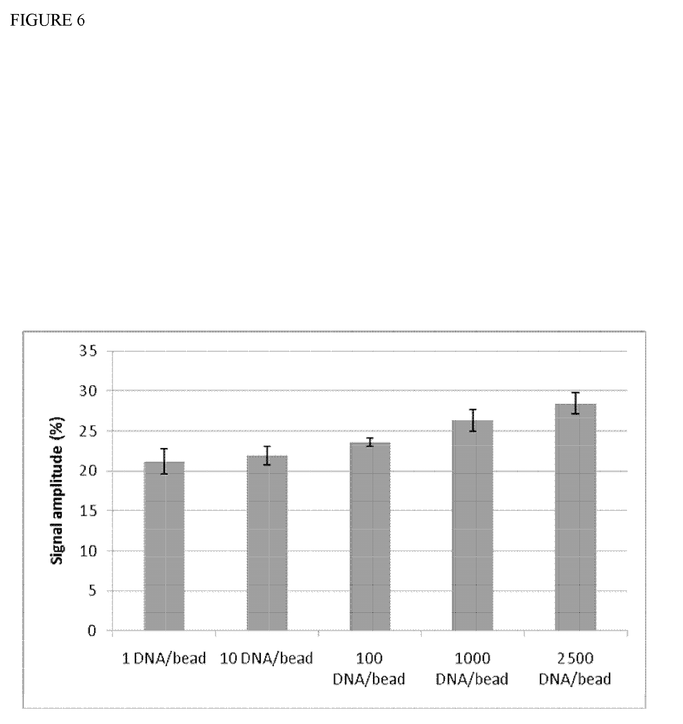

In particular, the present invention describes how the surface contact and thus the binding probability of magnetic nanoparticles can be increased by virtue of putting a repulsive surface structure on the particles. Surprisingly, it could be observed that repulsive forces either deriving from a high charge or a steric coating on the particles has two important effects. First, it increases the repulsion between the particles leading to a decrease of clustering of particles which is advantageous in terms of unspecific binding of particles to each other. The second important effect is that the repulsion leads to formation of an overall "pushing" force thus resulting in a pushing of the particles toward the surface without the need to increase the particle concentration. In an experiment as shown in Fig. 4 magnetic particles were functionalized with dsDNA of varying length and the charge on the particles by virtue of their zeta potential was measured. In an optomagnetic assay the inventors could observe that the surface contact of the particles increases with the charge of the particles. As can be seen in Fig. 5, the signal amplitude corresponding to the surface contact is proportional to the number of charged molecules attached to the particles. Hence, without wishing to be bound to a theory the increased surface contact leads to an increased reaction rate and finally to increased signal changes at the end of the assay (see, for example, Fig. 6). Based on these and other findings, which are further described in detail herein below, the inventors developed the concept of a repulsive surface architecture of magnetic nanoparticles suitable to generate repulsive forces and a pushing effect which advantageously augments the binding probability of magnetic nanoparticles. The concept can be implemented by a single type of magnetic particles comprising repulsive surface structures, or alternatively with a mixture of at least two types of magnetic particles, wherein one type is functionalized with a first binding molecule capable of specifically binding to said target molecule, wherein said first binding molecule is attached to the particle, and a second type is functionalized with a repulsive surface structure.

Accordingly, the present invention relates in a second aspect to a device for detecting a target molecule within a sample comprising a sample container for the measurement of the target molecule within a sample, a mixture of at least two types of magnetic particles, wherein one type is functionalized with a first binding molecule capable of specifically binding to said target molecule, wherein said first binding molecule is attached to the particle, and a second type is functionalized with a repulsive surface structure, which is directly attached to the surface of said particle, wherein said repulsive surface structure covers the surface of the magnetic particle so as to result in a specific net charge and/or steric pushing of the magnetic particle, and a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface.

In a preferred embodiment of the present invention said first binding molecule is attached to the particle via a linker molecule.

In another preferred embodiment of the present invention said linker molecule is longer than the repulsive surface structure.

In another preferred embodiment of the present invention said linker molecule is or comprises said repulsive surface structure.

In yet another preferred embodiment of the present invention said repulsive surface structure is further functionalized with a first binding molecule capable of specifically binding to said target molecule.

In another preferred embodiment of the present invention, said pushing effect is determined by an increase of the surface interaction by a least 1%.

In a further preferred embodiment of the present invention the repulsive surface structure as mentioned above is a charged structure or a steric coating.

In yet another preferred embodiment of the present invention said charged structure conveys an zeta potential to said magnetic particle of at least about 25 mV. measured under suitable conditions and/or said steric coating conveys a hydrodynamic radius of said magnetic particle of at least about 105 % of the radius of said magnetic particle.

In yet another preferred embodiment of the present invention said first binding molecule is an antibody or a fragment thereof, an aptamer, or a complementary nucleic acid.

In a further preferred embodiment of the present invention the charged structure is or comprises a nucleic acid molecule, a hydro-gel, or a polymer.

In a further particularly preferred embodiment of the present invention the nucleic acid molecule is selected from the group consisting of dsDNA, a PNA molecule, a PNA-DNA duplex, and an RNA-DNA duplex.

In yet another preferred embodiment of the present invention the linker molecule and/or said repulsive surface structure is not cleavable by DNase and/or a restriction enzyme capable of cutting a double stranded nucleic acid molecule.

In a further preferred embodiment of the present invention the nucleic acid molecule, preferably dsDNA, a PNA molecule, a PNA-DNA duplex, and an RNA-DNA duplex, has a length of about 50 -1000 nucleotides.

In a particularly preferred embodiment of the present invention the nucleic acid molecule, preferably dsDNA, a PNA molecule, a PNA-DNA duplex, and an RNA-DNA duplex has a length of about 100 nucleotides.

In a further aspect the present invention relates to a method of detecting the presence or amount of a target molecule within a sample comprising the steps of contacting the sample and a first binding molecule attached to a particle in a device as described herein and contacting the sample with a second binding molecule capable of attaching to a sensor surface wherein the first binding molecule and/or the second binding molecule are capable of specifically binding to said target molecule, and optionally a target analog molecule attached to the sensor surface; wherein the target molecule is capable of interfering with the binding of the first binding molecule to the target analog molecule, optionally adding magnetic particles comprising a repulsive surface structure as defined herein and detecting the number of particles bound to the sensor surface, wherein a magnetic force is applied to bring the particle into close proximity with the sensor surface; and wherein the number of bound particles is directly or inversely related to the amount of target molecules present in the sample.

In a further embodiment of the present invention the detection of bound particles occurs via frustrated total internal reflection (FTIR) or via measurement of scattered light from said bound particles near the surface.

In another aspect the present invention relates to the use of a magnetic particle as defined herein above or below for detecting a target molecule within a sample.

In a particularly preferred embodiment of the present invention the target molecule as mentioned in the context of the device, method or use as described herein above is cardiac troponin I (cTnI), NT-proBNP or parathyroid hormone.

BRIEF DESCRIPTION OF THE DRAWINGS

Fig. 1 shows the principle of FTIR detection. Light from a light source enters a cartridge, is reflected from the cartridge/fluid interface and imaged on a detector. If particles are present in the evanescent field, created on this interface, the reflected light intensity decreases.

Fig. 2 shows a sandwich immunoassay using Magnotech technology. In panel (1) magnetic particles coated with a primary antibody directed against the target disperse in the sample liquid and bind the target. In panel (2) top and bottom coils actuate the magnetic particles in a pulsed manner, resulting in binding to the sensor surface where a secondary antibody can bind to the bound target molecule. In panel (3) non-bound particles are removed from the sensor surface and bound particles are detected using an evanescent field.

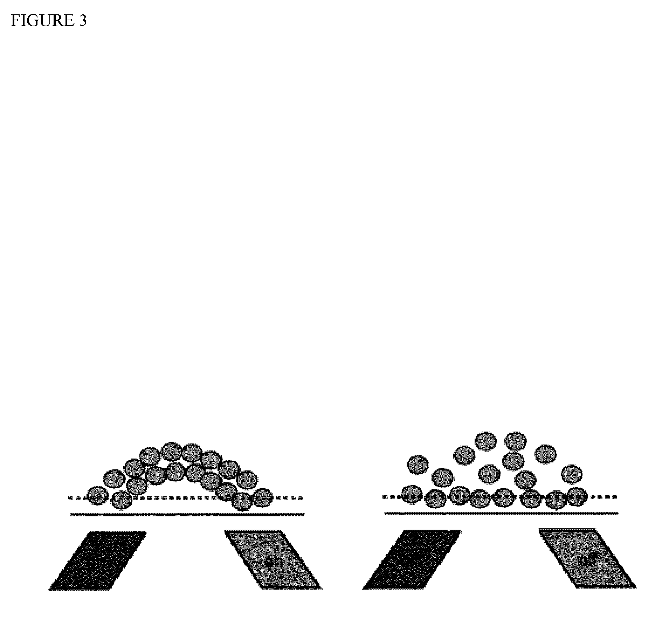

Fig. 3 shows magnetic particles with the magnets switched on or off. When the magnet is switched on (left), the particles form large strings. As a result, fewer particles are close enough to the surface to be detected (area indicated by dashed line). When the magnet is switched off (right), the particles can diffuse to the sensor surface, partly "pushed" by the top particles.

Fig. 4 shows measured zeta potential values for 500-nm Ademtech particles, functionalised with streptavidin molecules and bound to different amounts of biotin-functionalised dsDNA of varying lengths.

Fig. 5 shows screenshot of the biosensor software during a Magnotech FTIR assay. On the left side, the image of the evanescent field is depicted. The signal acquired in the different regions of interest is displayed as a function of time in the graph on the right. The arrow indicates the amplitude of the signal, which is a measure for the surface contact.

Fig. 6 shows FTIR signal amplitudes for assays performed with varying amounts of biotin-tagged 97bp dsDNA attached to 500 nm Ademtech superparamagnetic particles, functionalised with streptavidin proteins. The more DNA molecules per particle, the larger the surface contact.

Fig. 7 show as signal change at the end of an FTIR assay. First, a single 97bp dsDNA fragment carrying a biotin at one end of the molecule and a Texas-Red molecule at the other end of the DNA was bound to the streptavidin particle. Subsequently, similar 97 bp DNA molecules, but lacking the Texas-Red molecule, were added per particle to the indicated amount. All particles were tested in an FTIR assay with a printed spot of anti-Texas Red antibodies.

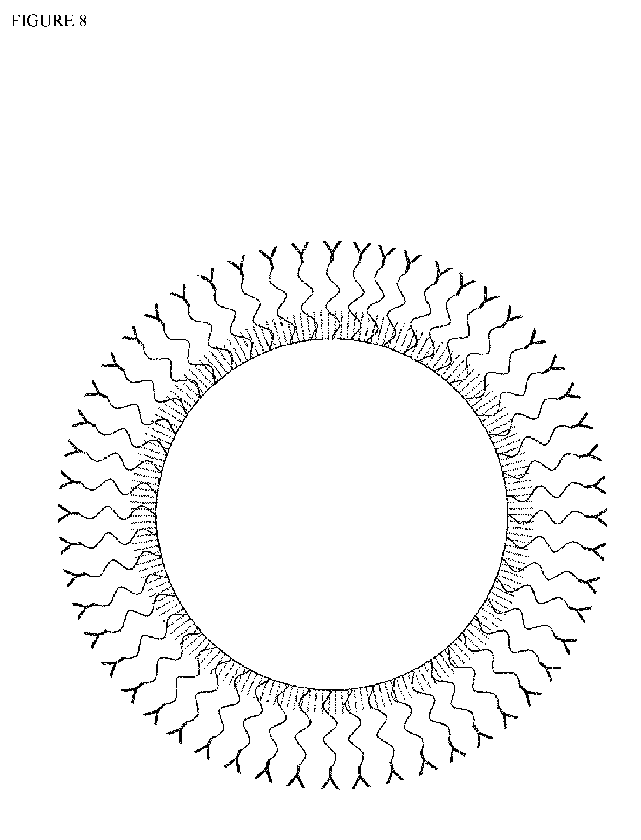

Fig. 8 shows a particle, which is functionalized with both short and long DNA fragments. The short DNA fragments (blue) carry a large charge, but are short enough to allow sufficient freedom of movement of the longer DNA fragments that carry a capture agent.

Fig. 9 shows measured hydrodynamic sizes (nm) and measured zeta potential values (mV) for 500-nm Ademtech particles, functionalised with streptavidin molecules and bound to different amounts of biotin-functionalised dsDNA of varying lengths.

DETAILED DESCRIPTION OF THE EMBODIMENTS

The present invention relates to a means and methods for detecting a target molecule within a sample. Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group, which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a device for detecting a target molecule within a sample comprising a sample container for the measurement of the target molecule within a sample, a magnetic particle, wherein said particle is functionalized with a first binding molecule capable of specifically binding to said target molecule, wherein said first binding molecule is attached to the particle, and a repulsive surface structure, which is directly attached to the surface of said particle, wherein said repulsive surface structure covers the surface of the magnetic particle so as to result in a specific net charge and/or steric repulsion of the magnetic particle, and a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface.

Envisaged by the present invention is a suitable device, preferably for detection of a target molecule using a biosensor system. Various analytical procedures to detect the presence and/or quantity of an analyte in a test sample or sample volume are known in the art. Typically, such a detection system may optically detect magnetic particles, which are functionalized with a suitable binding molecule in order to bind the analyte of interest. The presence or quantity of the analyte may be concluded from the number of analyte bound to the magnetic particles. Within the meaning of the present invention, the biosensor system comprising the device as described herein may be capable of detecting single magnetic particles and actuating the magnetic particles by a magnetic field generated by a magnetic field generator.

A "magnetic field generator" as used herein typically comprises one or more magnets within the optomagnetic device as described herein for generating a magnetic field with the sample chamber, wherein said magnetic field shall guide magnetic particles towards the contact surface. The magnetic field will typically have a nonzero gradient that allows to exert magnetic forces on magnetic (dipole) particles. Suitable examples of such magnetic field generators, or suitable systems comprising such magnetic field generators would be known to the person skilled in the art. In specific embodiments of the present invention the magnetic field generator may be a magnetic field generator as described in Bruls et al., Lab Chip, 2009, 9. 2504-3510, or be comprised in or be part of a system as described in Ranzoni et al., 2011, Nano Lett., 11, 2017-2022.

Typically, the magnetic particles can be actuated by applying a magnetic field such that the analytical procedure can be accelerated. In specific embodiments of the present invention, it is also envisaged that the use of a magnetic field may reduce the background signal due to removal of unspecifically bound particles.

Optomagnetic systems suitable for use in the present invention may comprise, for example, a biosensor cartridge comprising a sample container, a sensor device for sensing the magnetic particles, detection system, and a magnetic field generator. The systems may, in further embodiments, comprise one or more additional functional units such as a readout system, e.g. a screen or printer, an interface for database or computer systems, a calibration unit, a direct or indirect connectivity with high-throughput devices etc.

Also envisaged by the present invention are handheld devices for quick and instant analysis where a cartridge including the assay format may be inserted. Typically, such a device comprises a power supply, preferably in form of rechargeable batteries, a display, wireless connectivity such as WLAN for quick database access, or access to a laboratory information system. A typical biosensor system is, for example, described in Bruls et al., Lab Chip, 2009, 9. 2504-3510.

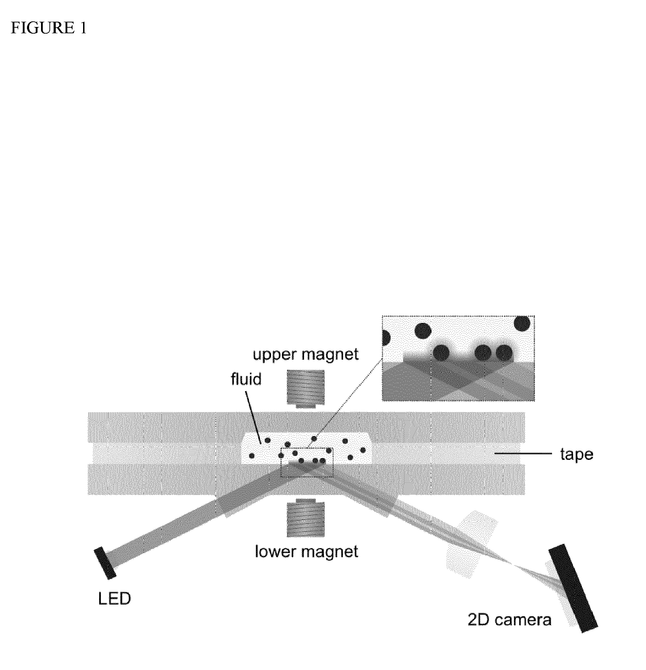

Particularly preferred are sensing devices based on an optical detection of magnetic particles. Without being limited thereto, an exemplary device is illustrated in Fig. 1 comprising a light source and a light detection system.

A "sample" as used herein refers to any sample, which includes a target molecule as defined herein. Such samples may, for example, include samples derived from or comprising stool, whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, fluid harvested from a site of an immune response, fluid harvested from a pooled collection site, bronchial lavage, urine, biopsy material, e.g. from all suitable organs, e.g. the lung, the muscle, brain, liver, skin, pancreas, stomach, etc., a nucleated cell sample, a fluid associated with a mucosal surface, hair, or skin. In addition, samples from environmental sources, e.g. water samples, meat or poultry samples, samples from sources of potential contamination etc. may be used.

The term "target molecule" as used herein refers to any molecule bound by a binding molecule and may for example be a biological substance such as a biomolecule, preferably a biomarker, complexes, cell fractions or cells. Preferably, a target molecule within the context of the present invention is a nucleic acid, e.g. DNA, or RNA molecule or an oligonucleotide such as a DNA or RNA oligonucleotide. Even more preferred are target molecules such as a peptide, a polypeptide or protein, a protein or polypeptide fragment, or functional protein or polypeptide domain, a drug molecule, a small molecule, or a vitamin.

A target molecule may be directly obtained from the samples as described herein above. In other situations samples may be subjected to sample preparation techniques, e.g. based on standard protocols, including, for example, partial purification, which renders the target molecules more accessible to binding partners, i.e. an affinity molecule as defined herein. For example, blood samples may be centrifuged to separate fractions including whole cells or membranes from serum, feces samples may be sectioned and homogenized with physiologically acceptable buffer and detergent, sputum samples may be liquefied and fractionated. Furthermore, antibiotics or bactericides may be added to samples to prevent further growth of any organisms present. Whole cells may also be removed or may be lysed to release their contents.

A "sample container" as used herein refers to a container made from any suitable material like glass, any transparent plastic, or a semiconductor in which the sample is measured. The magnetic particles as described herein may be already present in the sample container when the sample is introduced, may be introduced together with the sample, or may be introduced after the sample has been injected into the sample container. The sample container may further comprise a sensor surface comprising a second binding molecule. Preferably, the sensor surface is located at the bottom of the sample container. The sample container may, in specific embodiments, be located within an exchangeable cartridge, e.g. in a standalone component separate from the sensor device. Due to possible contamination with a sample, such a cartridge may preferably be a disposable item, made for instance from plastics by injection molding. Also envisaged are recyclable cartridges or recyclable cartridge parts, e.g. cartridges or cartridge parts, which can be cleansed or sterilized.

A "sensor surface" as used herein, defines an area at which an actual sensor event occurs or is detected. Typically, the sensor surface is located at the bottom of the sample container. The sensor surface may serve for the detection of the number of bound particles, which is directly or inversely related to the amount of target molecules present in the samples. Examples of such sensors surfaces and corresponding sensors which may be used in the context of the present invention are provided in Bruls et al., 2009, Lab Chip, 9, 3504-3510.

A "magnetic particle" as used herein means a small, localized object to which can be ascribed a physical property such as volume or mass. The term "magnetic particles" shall comprise both permanently magnetic particles as well as magnetisable particles, for example, superparamagnetic particles.

The term "superparamagnetic" as used herein describes a form of magnetism which appears in small ferromagnetic or ferromagnetic nanoparticles. It is known in the art that in sufficiently small nanoparticles, magnetization can randomly flip direction under the influence of temperature. The time between two flips is referred to as the Néel relaxation time. In the absence of an external magnetic field, when the time used to measure the magnetization of the nanoparticles is much longer than the Néel relaxation time, the magnetization appears to be in average zero, i.e. in the paramagnetic state. In such a state an external magnetic field is able to magnetize the nanoparticles similarly to a paramagnet. However, the magnetic susceptibility is much larger than those of paramagnets.

In particularly preferred embodiments of the present invention, the material, or particle, e.g. nanoparticle may be superparamagnetic particles, which are typically dispersed in aqueous solutions and retain a small charge, e.g. a negative or positive charge, or possess a certain zeta potential to ensure colloidal stability, keeping the particles separated and avoiding non-specific clustering.

Furthermore, a particle essentially behaves as a whole unit in terms of its transport and properties. Magnetic particles may accordingly be of a symmetrical, globular, essentially globular or spherical shape, or be of an irregular, asymmetric shape or form.

The size of a magnetic particle envisaged by the present invention typically ranges between 3 nm and 50 µm. Preferred are magnetic particles in the nanometer and micrometer range up to several micrometers. In particularly preferred embodiments the particle diameter is larger than 100 nm. The term "diameter" as used herein refers to any straight line segment that passes through the center of the particle and whose endpoints are on the particle surface. In case of non-spherical or semi spherical particles, the diameter is understood as the average diameter of the largest and shortest straight line segments that pass thought the center of the particle and whose endpoints are on the particle surface. It is further understood that a radius of a particle as defined herein is half of its diameter as defined herein above. Particularly preferred are magnetic nanoparticles, e.g. particles of a diameter of about 100 nm to 10 micrometer, more preferably 100 nm to 3 µm, even more preferably 300 nm to 1000 nm, e.g. 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, 500 nm, 510 nm, 520 nm, 530 nm, 540 nm, 550 nm, 560 nm, 570 nm, 580 nm, 590 nm, 600 nm, 620 nm, 650 nm, 670 nm, 700 nm, 720 nm, 750 nm, 770 nm, 800 nm, 820 nm, 850 nm, 870 nm, 900 nm, 920 nm, 950 nm, 970 nm, 1000 nm, or any value in between. Even more preferred are magnetic nanoparticles having a diameter of about 500 nm.

Preferably, the magnetic particles according to the present invention are functionalized with a first binding molecule. Envisaged by the present invention are also magnetic particles further comprising a repulsive surface structure as described herein.

The term "binding molecule" as used herein refers to any molecule having a high binding affinity for a second molecule, i.e. an interaction partner. A binding molecule in the sense of the present invention typically comprises binding or capture moieties capable of binding a specific target molecule as defined herein such as a biomolecule or a biomarker, or capable of binding a molecule-containing target entity, such as for example a virus, or a cell or a cell fragment, or material derived from tissue.

In a particularly preferred embodiment, the binding molecule is selected from the group consisting of aptamers, peptides, proteins, oligonucleotides, in particular complementary nucleic acids, and molecular imprinted polymers, ligands or receptors, and lectins, wherein the binding molecule preferably is an antibody or a fragment thereof or a complementary nucleic acid.

An "aptamer" as used within the context of a binding molecule may be a short nucleic acid molecule, e.g. an RNA, DNA, PNA, CNA, HNA, LNA or ANA molecule or any other suitable nucleic acid format known to the person skilled in the art, being capable of binding to a target molecule as defined herein, preferably to a nucleic acid target molecule as defined herein. Furthermore, the present invention envisages peptide aptamers, i.e. aptamers which are able to specifically bind to (a) protein(s), polypeptide(s) or peptide(s) comprising a specific amino acid sequence(s).

The term "PNA" relates to a peptide nucleic acid, i.e. an artificially synthesized polymer similar to DNA or RNA which is used in biological research and medical treatments, but which is not known to occur naturally. The PNA backbone is typically composed of repeating N-(2-aminoethyl)-glycine units linked by peptide bonds. The various purine and pyrimidine bases are linked to the backbone by methylene carbonyl bonds. PNAs are generally depicted like peptides, with the N-terminus at the first (left) position and the C-terminus at the right. While DNA and RNA have a desoxyribose and ribose sugar backbone, respectively, the PNA-backbone is composed of repeating N-(2-aminoethyl)-glycine units linked by peptide bonds. It is known in the art that PNA oligomers also show greater specificity in binding to complementary DNAs. Further details may be derived from any suitable literature source or textbook, e.g. Nielsen PE, Egholm M (1999), An Introduction to Peptide Nucleic Acid, Curr. Issues Mol. Biol. 1 (2): 89-104.

The term "CNA" relates to an aminocyclohexylethane acid nucleic acid. Furthermore, the term relates to a cyclopentane nucleic acid, i.e. a nucleic acid molecule comprising for example 2'-deoxycarbaguanosine.

The term "HNA" relates to hexitol nucleic acids, i.e. DNA analogues which are built up from standard nucleobases and a phosphorylated 1, 5-anhydrohexitol backbone.

The term "LNA" relates to locked nucleic acids. Typically, a locked nucleic acid is a modified and thus inaccessible RNA nucleotide. The ribose moiety of an LNA nucleotide may be modified with an extra bridge connecting the 2' and 4' carbons. Such a bridge locks the ribose in a 3'-endo structural conformation. The locked ribose conformation enhances base stacking and backbone pre-organization. This may significantly increase the thermal stability, i.e. melting temperature of the oligonucleotide.

The term "ANA" relates to arabinoic nucleic acids or derivatives thereof. A preferred ANA derivative in the context of the present invention is a 2'-deoxy-2'-fluoro-beta-D-arabinonucleoside (2'F-ANA).

Typically, (a) peptide aptamer(s) is/are a variable peptide loop(s), comprising for example 10 to 20 amino acids. In the context of the present invention the peptide aptamer(s) may in specific embodiments be attached at one or both ends to a scaffold structure. The scaffold structure may be any molecule, preferably a protein, e.g. a protein, which has good solubility properties. Suitable scaffold molecules would be known to the person skilled in the art. An example of suitable scaffold molecule to be used in the context of the present invention is the bacterial protein thioredoxin-A. The aptamer peptide loop may preferably be inserted within a reducing active site of the scaffold molecule. Alternatively, staphylococcal protein A and domains thereof and derivatives of these domains, such as protein Z or lipocalins may be used as scaffold structures in the context of the present invention. Nucleic acid or peptide aptamers may be generated according to any suitable method known to the person skilled in the art, e.g. via PCR or molecular synthesis approaches or yeast two-hybrid approaches.

A "peptide" as used within the context of a binding molecule may comprise or alternatively consist of a stretch of 2 to 35 amino acids, amino acid derivatives or a mixture thereof. The peptide may be linear, branched, circular or mixture thereof. A peptide affinity molecule may also be attached to a scaffold structure as defined herein above.

A "protein" as used within the context of binding molecule may comprise or alternatively consist of a stretch more than about 35 amino acids, amino acid derivatives or a mixture thereof. The protein may have a linear, branched, circular form or be comprised of a mixture of these forms. A protein binding molecule may also be attached to a scaffold structure as defined herein above.

An "oligonucleotide" as used within the context of a binding molecule may comprise or alternatively consist of a stretch of about 5 to 120 nucleotides, e.g. a stretch of 10, 20, 30, 40, 50, 60, 70, 80, 90 or 100 nucleotides, preferably of about 15 to 60 nucleotides. An oligonucleotide binding molecule may preferably be an RNA molecule or a DNA molecule, or a mixture of both.

Envisaged are binding molecules, which are complementary nucleic acid molecules. The term "complementary nucleic acid molecule" refers to a molecule of a defined sequence, where the single strands are complementary to each other. It is known in the art that complementary strands of a double stranded nucleic acid molecule have a strong affinity to each other due to the formation of base pairing. Also envisaged are single stranded oligonucleotide sequences, which are attached or integrated into the linker molecule structure as defined herein. In particularly preferred embodiments such suitable linker also comprise of consist a nucleic acid molecule. The single stranded stretch is capable of recognizing and hybridizing to the complementary nucleotide sequence of interest with high affinity. In such an analysis the target molecule comprises an oligonucleotide that is complementary or almost complementary to the binding molecule.

The term "molecular imprinted polymer" as used herein refers to a polymer, which was formed in the presence of a molecule that is extracted afterwards, leaving complementary cavities behind. Typically, a molecular imprinted polymer shows a certain chemical affinity for the original molecule. A molecular imprinted polymer may be composed of any suitable polymeric unit known to the person skilled in the art. Techniques for their production include polymerization techniques such as bulk, precipitation, emulsion, suspension, dispersion, gelation, and multi-step swelling polymerization. Particularly preferred are hierarchical imprinting methods.

An "antibody" as used within the context of a binding molecule refers to an immunoglobulin molecules and immunologically active portions of immunoglobulin molecules, i. e. molecules that contain an antigen binding site that immunospecifically binds an antigen. The immunoglobulin molecules of the invention can be of any type (e. g., IgG, IgE, IgM, IgD, IgA and IgY), class (e. g., IgG1, IgG2, IgG3, 1gG4, 1gA1 and IgA2) or subclass of immunoglobulin molecules. Antibodies of the present invention may be described or specified in terms of the epitope(s) or portion(s) of a target molecule, e.g. polypeptide of the present invention, which they recognize or specifically bind. Specific epitopes and their interaction with antibodies would be known to the person skilled in the art. The term "specifically binding" as used herein refers to the immunospecific detection and binding of an antibody to an antigenic epitope. The term "specifically binding" excludes non-specific binding but does not necessarily exclude cross-reactivity with other antigens, in particular with antigens comprising the same antigenic epitope detected by the present antibody.

The antibody may be a polyclonal, monoclonal, multispecific, human, humanized or chimeric antibody, single chain antibody, or constitute a Fab fragment, Fab' fragment, a fragment produced by a Fab expression library, F(ab')2, Fv, disulfide linked Fv, minibody, diabody, scFv, sc(Fv)2, whole immunoglobulin molecule, small modular immunopharmaceutical (SMIP), binding-domain immunoglobulin fusion protein, camelized antibody, $V_{HH}$ containing antibody, an anti-idiotypic (anti-Id) antibody an any epitope-binding fragment(s) of any of the above. Most preferably, the antibodies are human antigen-binding antibody fragments of the present invention and include Fab, Fab' and F (ab')2, Fv, single-chain Fvs (scFv), sc(Fv)2, single-chain antibodies, disulfide-linked Fvs (sdFv) and fragments comprising either a VL or VH domain.

The antibodies according to the invention may be from any animal origin including birds and mammals. Preferably, the antibodies are human, murine (e.g. mouse and rat), donkey, monkey, rabbit, goat, guinea pig, camel, horse, or chicken antibodies.

The antibodies according to the present invention may be monospecific, bispecific, trispecific or of greater multispecificity. Multispecific antibodies may be specific for different epitopes of a target molecule, e.g. a polypeptide according to the present invention or may be specific for both a target molecule, e.g. polypeptide according to the present invention as well as for a heterologous epitope, such as a heterologous polypeptide or solid support material. Preferred are monospecific antibodies.

The term "ligand" as used herein generally refers to a substance that forms a complex with a target molecule such as a biomolecule and can thus specifically bind to the molecule with a high binding affinity. In particular, a ligand may be a signal triggering molecule capable of binding to a site on a target protein. A ligand often binds to a binding partner often referred to as a receptor. Receptor-ligand binding systems for use in the detection of target molecules are known in the art. Typically, ligand binding to a receptor alters the chemical conformation, i.e. the three dimensional shape of the receptor protein. The conformational state of a receptor protein determines the functional state of a receptor. Suitable ligands within the context of the present invention may include, for example, enzyme substrates, enzyme inhibitors, receptor agonists and antagonists, activators such as DNA-binding proteins, or neurotransmitters.

The term "lectin" as used herein refers to a protein or glycoprotein capable of specific recognition of and reversible binding to carbohydrate moieties of complex glycoconjugates without altering the covalent structure of any of the recognized glycosyl ligands. Examples of suitable lectins within the context of the present invention include monovalent lectins like bacterial and plant toxins, which are able to bind to sugar moieties in cell walls or membranes. Other suitable lectins within the meaning of the present invention are mannose binding lectins, galactose/N-acetylgalactoseamine binding lectins, N-acetylglucosamine binding lectins, N-acetylneuraminic acid binding lectins or fucose binding lectins.

The term "first binding molecule" as used herein refers to a binding molecule as described herein, capable of specifically binding to a target molecule within a sample. The first binding molecule may be attached to the magnetic particle surface by any suitable method or in any suitable manner known to the person skilled in the art, for example via a linker molecule and/or a repulsive surface structure as defined hereinafter, or by a direct contact with the magnetic particle surface.

The term "attached to the particle" as used herein refers to the covalent or noncovalent binding or coupling of the first binding molecule to the magnetic particle. In particular, such a coupling may for example be a covalent binding, a van-der-Waals binding or an electrostatic binding between the layers. The attachment to the magnetic particle may be reversible or may be terminatable, e.g. by changing the pH, the temperature, the concentration of ions, by illuminating with light, by enzymatic degradation or enzymatic cleavage etc. The first binding molecule may, in certain embodiments, be attached to the particle via a linker structure, e.g. a linker molecule as defined herein. The first binding molecule may for this purpose be coupled to a linker molecule, which in turn is directly or indirectly attached to the particle surface. It is also conceivable that the first binding molecule is coupled to the repulsive structured as described herein. Also envisaged by the present invention is that the first binding molecule may be in direct contact with the magnetic particle surface.

In specific embodiments of the present invention the coupling, binding or attachment of the first binding molecule to the magnetic particle, or particle surface, or to the flat sensor surface may be a direct or indirect binding.

The term "direct binding" as used herein means that the first binding molecule has an immediate connection to the magnetic particle without the presence of intermediate, bridging or connector molecules or functionalities.

The term "indirect binding" as used herein means that the binding molecule is not directly attached to the surface of the magnetic particle, but can be indirectly linked via further intermediate, bridging or connector molecules such as, for example, other binding molecules, linker molecules or charged structures. For example, avidin, streptavidin derivatives, (strept)avidin, avidin-related proteins, avidin-like entities such as tamavidin 1 and 2, bradavidin, NeutrAvidin etc. may be used as connector between a magnetic particle, and an interacting molecule which comprises a compatible binding moiety such as biotin. In specific embodiment, a magnetic particle may be coated with or covered by an avidin or streptavidin connector. Further preferred examples of interaction couples useful as connector molecules are biotin/avidin, any antibody/antigen couple, e.g. anti FITC, FITC, anti-TexasRed/TexasRed, anti-digoxygenin/digoxygenin, and nucleic acid complementary strands as mentioned herein above. Envisaged by the present invention is in particular the use of nucleic acid complementary strands, because of the high degree of multiplexing due to the almost unlimited specific combinations. It will be also be appreciated by the person skilled in the art that such connector molecules which consist of or comprise a nucleic acid sequence can be easily integrated into a linker structure.

The term "repulsive surface structure" as used herein refers to a structure which may be directly or indirectly attached to the surface of a magnetic particle as described herein. It is preferred that the repulsive surface structure is directly attached to the surface of a magnetic particle, i.e. that it has an immediate connection to the magnetic particle without the presence of intermediate, bridging or connector molecules or functionalities.

A repulsive surface structure within the meaning of the present invention comprises molecules, polymers or and/or a mesh of molecules capable of conferring a repulsive force. The term "repulsive force" as used herein refers to forces, which lead to a repulsion of molecules or particles. Preferably envisaged by the present invention is the repulsion of magnetic particles. In general, a repulsive force between particles may be the result of interparticle forces such as excluded volume repulsion, electrostatic repulsion, entropic forces, or steric forces between polymer covered surfaces. The term "excluded volume repulsion" means the impossibility of any overlap between solid particles or, where applicable, between solid particles including surface structures present on the particles.

"Electrostatic repulsion" between particles is observed when particles carry a net electrical charge. If two particles carry the same net charge, i.e. either positive or negative charge they will repel each other. The term "entropic forces" refers to forces which are based on the second law of thermodynamics, describing the tendency of a system to progress to a state in which entropy is maximized. This can result in effective repulsive forces between solid spheres, e.g. magnetic particles. The term "steric repulsive force" is based on a steric effect. It is to be understood that on the atomic level, steric effects arise from the fact that each atom within a molecule occupies a certain amount of space. If atoms are brought to close to each other, the associated cost in energy is high due to overlapping electron clouds (Pauli or Born repulsion) and may affect the molecule's preferred shape or conformation and reactivity. Hence, steric hindrance or steric effects can be seen as repulsion between the electron clouds of the individual atoms and can in principal be defined as a result of electrostatic repulsion on the atomic level.

The wording "wherein said repulsive surface structure conveys an electrostatic and/or steric pushing effect on said magnetic particles towards said sensor surface" as used herein means that an entire ensemble of particles is pushed towards the sensor surface. This overall pushing effect is based on or caused by electrostatic pushing effects of repulsive surface structures of a magnetic particle, e.g. a electrostatic pushing effect between two or more particles, or steric pushing effects of repulsive surface structures of a magnetic particle, e.g. a steric pushing effect between two or more particles, or a combination of both, an electrostatic pushing effect and a steric pushing effect of magnetic particles, e.g. between two or more particles. The overall pushing effect may, for example, be dependent on the number of particles in the assay, the number of particle in the vicinity of the sensor surface, the overall charge of two or more particles, the proportion of electrostatic vs. steric pushing, and further parameter known to the person skilled in the art. These parameters may be adjusted and/or modified in accordance with specific needs and necessities of embodiments of the device or of assays carried out as described herein.

In a further preferred embodiment of the present invention the overall pushing effect as defined herein above is determined by measuring the increase of the surface interaction. The term "increase of the surface interaction" as used herein refers to an increase of the amount or number of particles being present at or close to the sensor surface as a result of the repulsion forces between magnetic particles according to the present invention. In other words, the increase of surface interaction thus means the increase of interaction of particles with the sensor surface. The increase of surface interaction can be measured via the amount of time the particles spend in close contact with the sensor surface. The term "close contact" means that the particles are near or at the surface so as to generate a signal when being close enough to the sensor surface, e.g. a light signal, using appropriate measurement methods. Suitable methods for the measurement of magnetic particles near or at the sensor surface are known in the skilled person and have been described, e.g. in Bruls et al., Lab Chip, 2009, 9. 2504-3510.

In preferred embodiments of the present invention the increase in surface interaction is determined by measuring the signal amplitude during the assay. Without being limited thereto, one envisaged example of determining the increase of surface interaction is the measurement of the signal amplitude via frustrated total internal reflection (FTIR) as described herein. The resulting FTIR signal amplitude then corresponds to the number of particles present in the evanescent field.

The increase of surface interaction is calculated from the signal amplitude resulting from the measurement using magnetic particles functionalized with a repulsive surface structure as defined herein above in relation to the signal amplitude resulting from the measurement using non-functionalized magnetic particles, i.e. without any repulsive surface structure. For instance, if the increase of the surface contact is increased by a factor of two, i.e. the signal amplitude using magnetic particles functionalized with a repulsive surface structure is double the signal amplitude of non-functionalized magnetic particles, the increase in the surface interaction is 100%.

In preferred embodiments of the present invention the increase of the surface interaction between particles and the sensor surface, may be at least about 1%. The increase of the surface interaction may, for example, be 1%, 2%, 3%, 4%, 5%, 6%, 7%, 8%, 9%, 10%, 15%, 20%, 25%, 30%, 35%, 40%, 45%, 50%, 60%, 70%, 80%, 90%, 100%, 120%, 140%, 160%, 180%, 200%, 220%, 240%, 260%, 280%, 300%, 320%, 340%, 360%, 380%, 400%, 420%, 440%, 460%, 480%, 500% or more than 500%.

In particularly preferred embodiments of the present invention a repulsive surface structure may be a charged structure or a steric coating present on the magnetic particle.

A "steric coating" as used herein refers to a surface structure, which may be present on a magnetic particle in the form of a coating, and which provides repulsion between magnetic particles. One important effect envisaged by the present invention is that the presence of such a steric coating will result in an increased mutual repulsion of the magnetic particles which repulsion in turn leads to the formation of a pushing force thus resulting in a pushing of the magnetic particles toward the flat sensor without the need to increase the magnetic particle concentration as described in the art (pushing effect). It is to be understood that the steric hindrance of such a steric coating generates a steric pushing effect as described herein. The envisaged approach has thus the effect that the surface contact of the magnetic particles increases with the repulsion force of the magnetic particles thus leading to an increased reaction rate and finally to increased signal changes at the end of the assay. It will be appreciated by the skilled person that the steric pushing effect can be measured by virtue of the hydrodynamic radius of the magnetic particles as described hereinafter.

The present invention describes one way of how such a steric pushing effect can be achieved, namely by providing a mesh of molecules on the magnetic particles surface which may act as a steric coating or barrier to prevent unspecific binding of unrelated molecules, or, which keeps other molecules or magnetic particles in a certain distance. It will be appreciated by the skilled person that magnetic particles having such a steric coating, e.g. in form of a coating comprising a mesh of molecules, may exert a certain steric repulsion.

The coating on the magnetic particles may be such that they form a network or mesh of molecules. Therefore, a layer of a coating according to the present invention may be comprised of small units or entities, which have identical or similar chemical, physical and/or biological properties. Preferably, a layer of the coating as described herein may comprise biological or chemical molecules capable of forming polymers of a certain length. Suitable polymers are, for instance, carbohydrates, lipids, polyethylene glycol, polysaccharides, dendrimers, dendrons, nanotubes, or a mixture thereof. Preferably, these polymeric entities are comprised in linkeror spacer molecules. In specific embodiments of the present invention, the coating may be comprised of a single surface layer or a multilayer shell structure.

The term "spacer molecules" as used herein refers to molecules, which primarily have the function of a spacer. For this purpose, these polymeric molecules have a certain length and strength in order to be able to act like polymeric fibers.

In specific embodiments of the present invention, the spacer molecules may have a length of up to about 500 nm, e.g. up to about 450, 400, 350, 300, 250 nm, 200, 150, 100, 90, 80, 70, 60, 50, 40, 30, 20, or 10 nm.

In further specific embodiments of the present invention, the spacer molecules may be linear, circular, or branched-like molecules, or a mixture thereof. If they are branched, they may be branched in different degrees, e.g. show 2, 3, 4, 4 or more hierarchical levels. The present invention further envisages a combination of different types, e.g. linear and branched, different branching degrees, different spacer lengths etc. of spacer molecules within a network, layer or shell structure. In alternative embodiments of the present invention the spacer molecule or mixture of spacer molecules provide an uniform mesh or network, i.e. an equispaced mesh or network with openings of essentially the same size. It is also envisaged that the mesh be provided with openings. It is in particular preferable if the mesh is so uniform, so that functional properties of the mesh with respect to the approachability of molecules and the motional freedom of molecules are uniform. These parameters may be adjusted by the lengths, branching degree etc. of the spacer molecules.

In further specific embodiments of the present invention, the spacer molecules can directly or indirectly be interlinked so as to form a three dimensional mesh of molecules. Such an interlinking may be based on functional groups at the termini of a molecule, or located in the center of a molecule, e.g. in case branching is envisaged. The control of interlinkage, its degree etc. may be implemented according to principles and methods known to the person skilled in the art, e.g. from a qualified textbook such as "Bioconjugate techniques", Hermanson, 2nd Ed. Academic Press, Elsevier or "Dendrimers and other dendritic polymers" Frechet and Tomalia, J. Wiley.

Suitable spacer molecules within the context of the present invention may be, for example, carbohydrates, lipids, polyethylene glycol (PEG), polysaccharides, dendrimers, dendrons, or nanotubes as defined herein, or any suitable derivatives or combinations thereof. The group of spacer molecules may further comprise hydrocarbon-based surfactants, cholesterol, glycolipids, bile acids, saponins, fatty acids, synthetic amphipathic block copolymers, natural products like egg yolk phospholipids.

A preferred example of a spacer molecule suitable for steric coatings is a polyethylene glycol (PEG) molecule. Polyethylene glycol molecules are capable of forming a dense and voluminous mesh of molecules on a surface. It will be also appreciated by the skilled person that PEG molecules can be interlinked to form a suitable steric coating as described herein above. Suitable molecules for interlinking of PEG molecules are connector molecules as described herein. The length may be made dependent on the envisaged overall thickness of the resulting coating layer, the envisaged length of the linker molecule, the envisaged flexibility, rigidity or stability of the linker molecule, and/or the envisaged number of structure molecules per magnetic particle.

Also envisaged by the present invention is the number of polyethylene glycol molecules attached on the magnetic particle. It is understood that the more molecules be attached to the magnetic particle the higher the density and thickness of the coating layer leading to a particle-particle repulsion through steric effect. It is thus understood that the steric repulsion of the magnetic particle is dependent of the thickness of the coating layer, which depends on the number of PEG molecules on the magnetic particles, the length of the PEG molecule and the number of bondages between the PEG molecules. The number of the molecules per particle may be at least about 10, 20, 30, 40, 50, 60, 70, 80, or 90, more preferably at least about 100, 150, 200, 250, even more preferably at least about 300, 350, 400, or 450, and most preferably at least about 500, or 1000.

In further embodiments of the present invention, the number of polyethylene glycol molecules as defined herein above attached to the magnetic particle may be varied. In specific embodiments, the number of the polyethylene glycol molecules per magnetic particle may be set to about 10, 20, 30, 40, 50, 60, 70, 80, or 90, more preferably to about 100, 150, 200, 250, even more preferably to about 300, 350, 400, or 450, and most preferably to about 500, 550, 600, 650, 700, 750, 800, 850, 900, 950, or 1000. In further embodiments, the number of the polyethylene glycol molecules per magnetic particle may be set to about 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 6000, 7000, 8000, 9000, 10000, 15000, 20000, 25000, 30000, 35000, 40000, 45000, 50000, 55000, 60000, 65000, 70000, 75000, 80000, 85000, 90000, 95000, 100000, 110000, 120000, 150000, or more. The number of polyethylene glycol molecules per magnetic particle may further be made dependent on the size or diameter of the magnetic particle, the area in which an attachment is possible or suitable, the ratio between the size or diameter of the magnetic particle and the length of the magnetic particle, the molecular identity of the polyethylene glycol molecules or any other suitable parameter known to the person skilled in the art.

In further embodiments, the surface coverage of a magnetic particle by polyethylene glycol molecules may be at about 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the overall surface of the magnetic particle or any value in between theses values.

In a further preferred embodiment a steric coating as defined herein conveys a hydrodynamic radius of a magnetic particle according to the present invention of at least about 105% of the radius of a magnetic particle as defined herein. The term "hydrodynamic radius of a magnetic particle" as used herein refers to the effective radius of a hydrated particle comprising a steric coating in solution, or the apparent size of the particle comprising a steric coating in solution. Preferably, a steric coating refers to a multitude, e.g. all, of the molecules contributing to the steric effect as described herein. The hydrodynamic radius of a particle comprising a steric coating as defined herein may be at least about 105% of the radius of a magnetic particle as defined herein, in particular of the radius of a magnetic particle which does not comprise such a steric coating. The hydrodynamic radius of a particle comprising a steric coating as defined herein may be, for example, about 105%, 110%, 120%, 125%, 130%, 140%, 150%, 160%, 170%, 180%, 190%, 200%, 250% or any value in between these values, or more, of the radius of a magnetic particle as defined herein, in particular of a magnetic particle which does not comprise such a steric coating.

In other particularly preferred embodiments of the present invention, the magnetic particles may comprise a charged structure in order to generate a repulsion force between the particles. The term "charged structure" as used herein refers to an entity, which contributes to the net charge of a magnetic particle according to the present invention. Charged structures according to the present invention may have a net charge that can be either positive or negative. It is understood that a charged structure is not limited to a specific type or form of molecules. A charged structure may, for example, comprise a polymer of one type of molecules or of two or more different types of molecules, a complex of different molecules or entities, or a compound comprising several molecules having different charges, e.g. different positive charges, different negative charges, or (different) positive and negative charges, resulting in a positive or negative overall net charge. Tools and methods for charge calculation are known to the skilled person, e.g. from Dewar, M.J.S., The Molecular Orbital Theory of Organic Chemistry, McGraw-Hill, and Inc., 1969; or Stewart, R., The Proton: Applications to Organic Chemistry, Academic Press, Inc., 1985, 72.

In specific embodiments of the present invention, the charged structure may be attached to the magnetic particle directly or indirectly so as to result in a specific net charge of the magnetic particle. The specific net charge can be either positive or negative. It is also conceivable that different charged structures are attached on the same magnetic particle to result in a mixture of charged structures. Within the context of the present invention a charged structure may cover the surface of the magnetic particle so as to provide an overall charged molecule having a specific net charge. It is known to the skilled person that the net charge of a nanoparticle can be determined, for example, via the measurement of the zeta-potential.

One envisaged advantage is that the increased repulsion due to the presence of increased charges on the magnetic particle leads to increased electrostatic repulsion, which in turn minimizes non-specific clustering of the magnetic particles. Reduction of non-specific clustering may help to reduce the background level without the use of a surfactant thus leading to improvement of the blank level in optomagnetic detection assays as described herein and thus improves the limit of detection. Another important effect envisaged by the present invention is that the presence of an increased charge on the magnetic particles, being either a negative or a positive charge, will result in an increased mutual electrostatic repulsion of the magnetic particles which in turn leads to the formation of an electrostatic "pushing" force thus resulting in a pushing of the magnetic particles toward the flat sensor without the need to increase the magnetic particle concentration as described in the art. The envisaged approach has the effect that the surface contact of the magnetic particles may increase with the charge of the magnetic particles, thus leading to an increased reaction rate and finally to increased signal changes at the end of the assay.

In further preferred embodiments, the increased presence of a charge on the magnetic particles can be achieved either by providing a linker molecule as described herein comprising or consisting of a charged structure or by providing a charged structure attached to the magnetic particles in addition to a linker molecule.

In specific embodiments of the present invention charged structures may cover the entire surface of a magnetic particle so to as to provide an overall charged molecule having a specific net charge. In alternative embodiments, charged structures may cover only portions, areas or sectors of a magnetic particle, e.g. 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% or less of the overall surface of a magnetic particle, or any value in between theses values.

In further embodiments of the present invention, the number of charged structure molecules attached to the magnetic particle may be varied, e.g. in accordance with an envisaged overall net charge of the magnetic particle. It is understood that the overall net charge of the magnetic particle varies, e.g. increases or decreases with the number of charged molecules being attached to the magnetic particle. The zeta potential (mV) may accordingly increase (negatively) with increasing length of the molecules.

It is thus conceivable that the sum of charges present on a magnetic particle contributes to the overall net charge. It is further appreciated that the overall net charge of the magnetic particle is a function of, inter alia, the number of molecules present on the magnetic particle and the length of the molecule, their positive or negative charge etc. In specific embodiments, the number of the charged structure molecules per magnetic particle may be set to about 10, 20, 30, 40, 50, 60, 70, 80, or 90, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 5000, 6000, 7000, 8000, 9000, 10000,15000,20000,25000,30000,35000,40000,45000 ,50000,55000,60000,65000, 70000, 75000, 80000, 85000, 90000, 95000, 100000, 110000, 120000, 150000, or more. The number of the charged structure molecules per magnetic particle maybe different or be made dependent on the size or diameter of the magnetic particle, the area in which an attachment is possible or suitable, the ratio between the size or diameter of the magnetic particle and the length of the magnetic particle, the molecular or chemical identity of the charged structure molecules, the envisaged use, e.g. assay, or any other suitable parameter known to the person skilled in the art.

Suitable charged structures in order to obtain this electrostatic pushing effect as described herein may comprise a biological or chemical structure or compound capable of contributing to a specific net charge such as a nucleic acid, a ribonucleic acid, a peptide, a polypeptide or protein, a carbohydrate, a lipid, or a polysaccharide, or any suitable derivatives or combinations thereof. Also envisaged are a hydro-gel, and a polymeric charged structures. Preferred is the use of nucleic acid based charged structure molecules.

"Nucleic acid based charged structure molecules" may be any nucleic acid molecules, or derivate or analog thereof. Such charged structure molecules may, for example, comprise single and/or double stranded DNA or RNA molecules, or any type of derivative thereof. Such charged structure molecules may further comprise, or consist of a PNA, CNA, HNA, LNA or ANA molecule as defined herein above, or any mixture or combination thereof, e.g. a combination of any one of DNA, RNA, PNA, CNA, HNA, LNA and ANA or a mixture of LNA nucleotides with DNA or RNA bases, or any mixture or combination with any other charged structure molecule as defined herein. In the context of nucleic acid based charged structures nucleic acids or analogs thereof may additionally be provided with chemical groups or units which carry a charge. For example, phosphate groups, charged nitrogen derivatives or charged sulfur derivatives may be included or added. Furthermore, charges may be accumulated by providing duplex molecules of which at least one molecule comprises charges, e.g. a duplex comprising DNA.

In particularly preferred embodiments of the present invention, the magnetic particles may be functionalized with charged structures, e.g. single stranded or double stranded nucleic acid, in particular dsDNA, which may have a varying length. The charge on the magnetic particles may accordingly be measured by virtue of their zeta potential. Envisaged by the present invention is that the surface contact of the magnetic particles increases with the charge of the magnetic particles thus leading to an increased reaction rate and finally to increased signal changes at the end of the assay. In certain embodiments of the present invention, a charged structure such as a nucleic acid may be present in a double-stranded or duplex form, i.e. comprising a strand and a complementary or anti-parallel counter-strand of a nucleic acid molecule associated by base pairing between the strands, or a sense or antisense-strand of a nucleic acid molecule. Alternatively, the charged structure may comprises a single-stranded nucleic acid or a single stranded ribonucleic acid molecule.

In a particular preferred embodiment of the present invention the charged structure comprises a molecule selected from the group consisting of a double stranded or dsDNA, double stranded or dsRNA, PNA, a PNA-DNA duplex, and a RNA-DNA duplex. One advantageous aspect, which is also envisaged by further preferred embodiments of the present invention, is that PNAs and PNA/DNA or PNA/DNA duplexes are not easily recognized by either nuclease or proteases making them resistant to enzymatic degradation.

In further embodiments of the present invention suitable nucleic acid based charged structure molecules may also comprise or consist of single stranded DNA molecules of different base compositions and/or lengths.

The single stranded molecules may, for example, encompass the repetition of base sequence, be entirely random, or be derived from nature, or be of only one base.

The nucleic acid based charged structure molecules or combinations thereof as defined herein above may be of different base compositions and/or lengths. The length of the molecule may vary between about 5 nucleotides to about 5000 nucleotides, preferably between about 50 nucleotides to about 3000 nucleotides, between about 100 nucleotides to about 2000 nucleotides even more preferably, most preferably between about 400 nucleotides to about 1000 nucleotides. Also envisaged are other lengths or any length value within the indicated range. The molecule may, for example, have a length of about 10, 20, 30, 40, 50, 60, 70, 80, 90, 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000nucleotides or more, or any number of nucleotides in between the mentioned values.

The length of the nucleic acid based charged structure molecules may be made dependent on the envisaged overall specific net charge, the envisaged length of the linker molecule, the envisaged flexibility, rigidity or stability of the linker molecule, and/or the envisaged number of charged structure molecules per magnetic particle. Suitable methods for the calculation of net charges would be known to the person skilled in the art, or can be derived from suitable literature sources.

In further embodiments of the present invention, the number of nucleic acid based charged structure molecules as defined herein above attached to the magnetic particle may be varied, e.g. in accordance with an envisaged overall net charge of the magnetic particle. It is understood that the overall net charge of the magnetic particle varies, e.g. increases or decreases with the number of nucleic acid based charged structure molecules being attached to the magnetic particle. In specific embodiments, the number of the nucleic acid based charged structure molecules per magnetic particle may be set to about 10, 20, 30, 40, 50, 60, 70, 80, or 90, more preferably to about 100, 150, 200, 250,even more preferably to about 300, 350, 400, or 450, and most preferably to about 500, 550, 600, 650, 700, 750, 800, 850, 900, 950, or 1000. In further embodiments, number of the nucleic acid based charged structure molecules per magnetic particle may be set to about 1100, 1200, 1300, 1400, 1500, 1600, 1700, 1800, 1900, 2000, 2100, 2200, 2300, 2400, 2500, 2600, 2700, 2800, 2900,3000,3100,3200,3300,3400,3500,3600,3700,3800,3900,4000,4100,4200,4300, 4400, 4500, 4600, 4700, 4800, 4900, 5000, 6000, 7000, 8000, 9000, 10000, 15000, 20000, 25000, 30000, 35000, 40000, 45000, 50000, 55000, 60000, 65000, 70000, 75000, 80000, 85000, 90000, 95000, 100000, 110000, 120000, or 150000. The number of nucleic acid based charged structure molecules per magnetic particle may further be made dependent on the size or diameter of the magnetic particle, the area in which an attachment is possible or suitable, the ratio between the size or diameter of the magnetic particle and the length of the magnetic particle, the molecular identity of the nucleic acid based charged structure molecules or any other suitable parameter known to the person skilled in the art. In further embodiments, the surface coverage of a magnetic particle by nucleic acid based charged structure molecules may be at about 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the overall surface of the magnetic particle or any value in between theses values.

Peptide molecules, polypeptide molecules or protein molecules, e.g. as defined herein above, may also be or comprise suitable charged structures due to the presence of charged entities within the amino acids. It is described in the art that the amine and carboxylic acid functional groups found in amino acids allow them to have amphiprotic properties. Carboxylic acid groups ($-CO_2H$) can be deprotonated to become negative carboxylates ($-CO_2^-$), and alpha-amino groups ($NH_2-$) can be protonated to become positive alpha-ammonium groups ($^+NH_3-$). The skilled person also knows that at pH values greater than the $pK_a$ of the carboxylic acid group the negative carboxylate ion predominates. At pH values lower than the $pK_a$ of the alpha-ammonium group the nitrogen is predominantly protonated as a positively charged alpha-ammonium group. The skilled person is further aware of the fact that the net charge of an amino acid depends on the pH and the $pK_a$ value. Tools and methods to calculate the net charge of an amino acids, peptides, polypeptides or proteins would be known to the person skilled in the art or can be derived from qualified literature references such as the Compute pI/Mw tool which allows the computation of the theoretical pI (isoelectric point) and Mw (molecular weight) for a list of UniProt Knowledgebase (Swiss-Prot or TrEMBL) entries or for user entered sequences (Gasteiger E., et al.;Protein Identification and Analysis Tools on the ExPASy Server; (In) John M. Walker (ed): The Proteomics Protocols Handbook, Humana Press (2005).

Such peptide, polypeptide or protein charged structures may be of different amino acid compositions and/or lengths. The length may be made dependent on the envisaged overall specific net charge, the envisaged length of the linker molecule, the envisaged flexibility, rigidity or stability of the linker molecule, and/or the envisaged number of charged structure molecules per magnetic particle. It is understood that the overall net charge of the magnetic particle is a function of the number of protein or peptide molecules on the magnetic particle and the length of the molecule.

In further embodiments of the present invention, the number of charged peptide, polypeptide or protein molecules as defined herein above attached to the magnetic particle may be varied, e.g. in accordance with an envisaged overall net charge of the magnetic particle. In specific embodiments, the number of the charged peptide, polypeptide or protein molecules per magnetic particle may be set to about 10, 20, 30, 40, 50, 60, 70, 80, or 90, more preferably to about 100, 150, 200, 250, even more preferably to about 300, 350, 400, or 450, and most preferably to about 500, 550, 600, 650, 700, 750, 800, 850, 900, 950, or 1000. In further embodiments, the number of the charged peptide, polypeptide or protein molecules per magnetic particle may be set to about 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 6000, 7000, 8000, 9000, 10000, 15000, 20000, 25000, 30000, 35000, 40000, 45000, 50000, 55000, 60000, 65000, 70000, 75000, 80000, 85000, 90000, 95000, 100000, 110000, 120000, 150000, or more. The number of charged peptide, polypeptide or protein molecules per magnetic particle may further be made dependent on the size or diameter of the magnetic particle, the area in which an attachment is possible or suitable, the ratio between the size or diameter of the magnetic particle and the length of the magnetic particle, the molecular identity of the charged polypeptide or protein molecules or any other suitable parameter known to the person skilled in the art. In further embodiments, the surface coverage of a magnetic particle by charged peptide, polypeptide or protein molecules may be at about 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the overall surface of the magnetic particle or any value in between theses values.

A charged structure may, in further embodiments, also be or comprise a carbohydrate molecule, e.g. as defined herein above, which comprises a charged entity capable of contributing to an overall net charge on the magnetic particle. Such carbohydrates may be of different compositions and/or lengths. The length may be made dependent on the envisaged overall specific net charge, the envisaged length of the linker molecule, the envisaged flexibility, rigidity or stability of the linker molecule, and/or the envisaged number of charged structure molecules per magnetic particle.

In further embodiments of the present invention, the number of charged carbohydrate molecules as defined herein above attached to the particle may be varied, e.g. in accordance with an envisaged overall net charge of the magnetic particle. In specific embodiments, the number of the charged carbohydrate molecules per magnetic particle may be set to about 10, 20, 30, 40, 50, 60, 70, 80, or 90, more preferably to about 100, 150, 200, 250, even more preferably to about 300, 350, 400, or 450, and most preferably to about 500, 550, 600, 650, 700, 750, 800, 850, 900, 950, or 1000. In further embodiments, the number of the charged carbohydrate molecules per magnetic particle may be set to about 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 6000, 7000, 8000, 9000, 10000, 15000, 20000, 25000, 30000, 35000, 40000, 45000, 50000, 55000, 60000, 65000, 70000, 75000, 80000, 85000, 90000, 95000, 100000, 110000, 120000, 150000, or more. The number of charged carbohydrate molecules per magnetic particle may further be made dependent on the size or diameter of the magnetic particle, the area in which an attachment is possible or suitable, the ratio between the size or diameter of the magnetic particle and the length of the magnetic particle, the molecular identity of the charged carbohydrate molecules or any other suitable parameter known to the person skilled in the art. In further embodiments, the surface coverage of a magnetic particle by charged carbohydrate molecules may be at about 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the overall surface of the magnetic particle or any value in between theses values.

A suitable charged structure may further be any lipid molecule, e.g. as defined herein above, which comprises a charged entity capable of contributing to an overall net charge on the magnetic particle. The charge in lipids, especially phospholipids is typically determined by the charge of the headgroup. Preferred examples of suitable headgroups capable of conferring a net charge of the lipid molecule are headgroups selected from the group consisting of phosphatidylcholine (PC), phosphatidylserine (PS), phosphatidyl ethanolamine (PE) and phophatidyglycerol (PG), or any combination thereof.

Such lipids may be of different compositions and/or lengths. The length may be made dependent on the envisaged overall specific net charge, the envisaged length of the linker molecule, the envisaged flexibility, rigidity or stability of the linker molecule, and/or the envisaged number of charged structure molecules per magnetic particle.

In further embodiments of the present invention, the number of charged lipid molecules as defined herein above attached to the magnetic particle may be varied, e.g. in accordance with an envisaged overall net charge of the magnetic particle. In specific embodiments, the number of the charged lipid molecules per magnetic particle may be set to about 10, 20, 30, 40, 50, 60, 70, 80, or 90, more preferably to about 100, 150, 200, 250, even more preferably to about 300, 350, 400, or 450, and most preferably to about 500, 550, 600, 650, 700, 750, 800, 850, 900, 950, or 1000. In further embodiments, the number of the charged lipid molecules per magnetic particle may be set to about 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 6000, 7000, 8000, 9000, 10000, 15000, 20000, 25000, 30000, 35000, 40000, 45000, 50000, 55000, 60000, 65000, 70000, 75000, 80000, 85000, 90000, 95000, 100000, 110000, 120000, 150000, or more. The number of charged lipid molecules per magnetic particle may further be made dependent on the size or diameter of the magnetic particle, the area in which an attachment is possible or suitable, the ratio between the size or diameter of the magnetic particle and the length of the magnetic particle, the molecular identity of the charged lipid molecules or any other suitable parameter known to the person skilled in the art. In further embodiments, the surface coverage of a magnetic particle by charged lipid molecules may be at about 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the overall surface of the magnetic particle or any value in between theses values.

A "hydrogel" comprising a charged structure refers to three dimensional of a network of polymer chains that are hydrophilic, sometimes found as a colloidal gel in which water is the dispersion medium. Typically, hydrogels are highly absorbent, i.e. they can contain over 99.9% water, natural or synthetic polymers and is able to retain large amounts of water with conservation of the network structure. Hydrogels may also possess a degree of flexibility very similar to natural tissue, due to their significant water content. As a result of the high water content, hydrogels are generally considered as biocompatible materials, which makes them particularly interesting for biomedical and pharmaceutical applications. Common ingredients of hydrogels may comprise, for example, polyvinyl alcohol, sodium polyacrylate, acrylate polymers and acrylate copolymers with an abundance of hydrophilic groups. Also envisaged are natural hydrogel materials such as materials including agarose, methylcellulose, hyaluronan and other naturally derived polymers. It is further preferred that hydrogels additionally comprise, comprise or consist of charged elements such as charged polymers or any other charged molecules or moieties, which may be used as charged coating of the magnetic particle surface. A homogenous hydrogel structure may advantageously contribute to an overall net charge of the magnetic particle leading to electrostatic repulsion of the magnetic particles. In a specific embodiment, the linker molecules and other components of the coating or surface structure may be embedded within an aqueous hydrogel framework system, which may further provide a certain stability of the molecules. The skilled person would know how to select suitable charged polymers in order to obtain a hydrogel with an overall net charge. In addition, means and methods for producing suitable hydrogels and attaching these on the magnetic particle surface would be known in the art. The hydrogels as described herein above may also comprise a polymeric charged molecule as described herein.

A charged structure may, in further embodiments, also be a "polymeric charged molecule". The term "polymeric charged molecule" or "charged polymer" as used herein refers to a ",polymer or copolymer comprising a plurality of repeating units selected from negatively or positively charged repeating units. Such a polymer or copolymer thus works as a polyelectrolyte. It is understood that the charge may be derived from negatively or positively charged groups within the repeating unit. Particularly preferred is a positively charged group selected from the group consisting of a quaternary ammonium group, a primary amine group, a secondary amine group, a tertiary amine group, a quaternary phosphonium group, a tertiary phosphonium group, an amide group, a heteroaromatic nitrogen group, and a sulfonium group.

The resulting positively charged polymers are also termed cationic polymers. The positive charge of a polymeric charged molecule may advantageously prevent the formation of coiled polymers. This allows them to contribute more to viscosity in their stretched state, because the stretched-out polymer takes up more space.

Preferred negatively charged polymers include, but are not limited to, a sulfate ester group, a carboxylate ester group, a phosphate ester group, a sulfone group, a sulfide group, a disulfide group, an ortho ester group, an anhydride group, and a beta-ketosulfone group, or any combination thereof.

Polyelectrolytes have been utilized in the formation of new types of materials known as polyelectrolyte multilayers (PEMs). These thin films are constructed using a layer-by-layer (LbL) deposition technique. During LbL deposition, a suitable growth substrate (usually charged) is dipped back and forth between dilute baths of positively and negatively charged polyelectrolyte solutions. During each dip a small amount of polyelectrolyte is adsorbed and the surface charge is reversed, allowing the gradual and controlled build-up of electrostatically cross-linked films of polycation-polyanion layers. It will be appreciated that such polyelectrolyte multilayers are suitable to provide magnetic particles of the present invention with an overall positive or negative net charge.

In a further preferred embodiment a charged structure as defined herein conveys a zeta potential to a magnetic particle according to the present invention of at least about 25 mV measured under suitable conditions. The skilled person knows that the absolute net charge of a particle can be determined by the measurement of the zeta potential. The term "absolute net charge" as used herein refers to the net or overall charge of a particle without indication of its positive or negative sign. As used herein, "zeta potential" or "ξ-potential" a measure for the electrokinetic potential in colloidal systems. The zeta potential can be expressed as the electrical potential in the interfacial double layer (DL) at the location of the slipping plane versus a point in the bulk fluid away from the interface. In other words, zeta potential is the potential difference between the dispersion medium and the stationary layer of fluid attached to a dispersed particle. The zeta potential indicates the degree of repulsion between adjacent, similarly charged particles in a dispersion. Suitable means and methods to determine the zeta potential of nanoparticles, e.g. magnetic nanoparticles, would be known to the skilled person, or can be derived from suitable literature sources. Zeta potential is not measurable directly but it can be calculated using theoretical models and an experimentally determined electrophoretic mobility or dynamic electrophoretic mobility. The term "measured under suitable conditions" means that the zeta potential is measured in relation to the dispersion medium. It is to be understood that the measurement is carried out under specific conditions of the dispersion medium. In specific embodiments of the present invention the zeta potential is determined at a specific buffer concentration. The skilled person is aware of suitable buffers for the dispersion of the magnetic particles and for measurement of the zeta potential. In particular preferred embodiments of the present invention the zeta potential is measured using a phosphate buffer having a concentration of 1mM, 5mM, 10mM, 15 mM, 20 mM, or 50 mM, or any value in between. The zeta potential may preferably be determined at a concentration of 10 mM phosphate buffer. In further specific embodiments the zeta potential is determined in a medium having a specific pH. Preferably, medium has a pH value of 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, or 8.5. It is particularly preferred that the zeta potential be determined at pH 7.4. In a particular preferred embodiment the zeta potential may preferably be determined at specific conditions, e.g. at a specific buffer concentration and a specific pH. It is most preferred that the zeta potential be determined at a concentration of 10 mM phosphate buffer, and at pH 7.4.

Accordingly, a charged structure may convey zeta potential of about +25mV or -25mV. The term "a charged structure" refers in this context to one charged structures, or preferably a multitude of charged structures, e.g. all charged structures, on a particle, as defined herein.

In specific embodiments, the zeta potential of a particle comprising a charged structure as defined herein may be about 25 mV, 30 mV, 35 mV, 40 mV, 45 mV, 50mV, 60 mV, 70 mV, 80 mV, 90 mV, 100 mV or any value in between, or more than 100 mV.

In further embodiments of the present invention, a charged structure as defined herein, in particular a multitude of charged structures, e.g. all charged structures, on a particle may convey an increase of zeta potential of said particle of at least 1 mV in comparison to the zeta potential of a particle without said charged structures. The increase of zeta potential of said particle in comparison to the zeta potential of a particle without said charged structures may be, for example, about 1 mV, 2 mV, 3 mV, 4 mV, 5 mV, 6 mV, 7 mV, 8 mV, 9 mV, 10 mV, 15 mV, 20 mV, 25 mV, 30 mV, 35 mV, 40 mV, 50 mV or any value in between these values, or more than 50 mV.

In yet another embodiment of the present invention, a charged structure as defined herein, in particular a multitude of charged structures, e.g. all charged structures, on a particle, may convey an increase of zeta potential of the particle by at least 5% in comparison to the zeta potential of a particle without said charged structures. The increase of zeta potential of said particle in comparison to the zeta potential of a particle without said charged structures may be, for example, about 5%, 7%, 10%, 12%, 15%, 20%, 25%, 30%, 35%, 40%, 50%, 55%, 60%, 70%, 80%, 100% or any value in between these values, or more than 100%.

The term "second binding molecule" as used herein refers to a binding molecule, which is directly or indirectly attached to the sensor surface as described herein. The second binding molecule may be the same type of molecule as the first binding molecule or may be a different molecule. In preferred embodiments of the present invention first and second binding molecule molecules are the same molecules. In specific embodiments of the present invention the second binding molecule is an antibody or a fragment thereof, preferably an antibody as defined herein above. In particular preferred embodiments of the present invention the second binding protein is capable of specifically recognizing and binding to the target molecule within a sample however, at a different binding site or epitope of the target molecule than recognized by the first binding molecule. In a further preferred embodiment, the second binding molecule may be a second antibody, e.g. an antibody as defined herein above, which recognizes a first binding molecule, e.g. a first antibody, which may be an antibody as defined herein above.

In a preferred embodiment of the present invention the first binding molecule is attached to the magnetic particle via a linker molecule.

In further preferred embodiments the second binding molecule may be attached to sensor surface via a linker molecule as described herein.

The term "linker molecule" as described herein can be any structure suitable to provide the attachment of the first binding molecule to the surface. The skilled person would be aware of means and methods for coupling a linker molecule to a magnetic particle surface. For this purpose the surface of the magnetic particle and/or the linker molecule may comprise an anchoring moiety capable of coupling a linker to a surface.

For this purpose the surface and/or the linker molecule may comprise an anchoring moiety capable of coupling a linker to a surface. The "anchoring moiety" as used herein is to be understood as a connector in between a linker molecule or one or more repulsive structure surface molecules and a surface. Of particular relevance is thus any bridging or connector molecule as described herein above. For example, avidin, streptavidin derivatives, (strept)avidin, avidin-related proteins, avidin-like entities such as tamavidin 1 and 2, bradavidin, NeutrAvidin etc. may be used for anchoring of a linker molecule and a surface or surface structure as described herein. For instance, the anchoring moiety may comprise biotin, while the surface is coated or functionalized with streptavidin. Means and methods for coating a surface with streptavidin are known to the skilled person, or can be derived from qualified textbooks or literature sources.

Further preferred examples of interaction couples suitable as connector molecules for anchoring are biotin/avidin, any antibody/antigen couple, e.g. anti FITC, FITC, anti-TexasRed/TexasRed, anti-digoxygnenin/digoxygenin, nucleic acid complementary strands as mentioned herein above. The anchoring moiety may also comprise nucleic acid complementary strands, because of the high degree of multiplexing due to the almost unlimited specific combinations. It will be also appreciated by the person skilled in the art that such connector molecules, which consist of a nucleic acid sequence can be easily integrated into a linker structure.

Also envisaged by the present invention are anchoring moieties comprising or consisting of a chemical group, which may be covalently linked (cross-linked) by coupling chemistry to a surface or a molecule bound to a surface. Crosslinking is the process of chemically joining two or more molecules by a covalent bond often referred to as bioconjugation. Typically, crosslinking reagents (or crosslinkers) are molecules that contain two or more reactive ends capable or chemically attaching to specific functional groups on proteins or other molecules such as protein functional groups, e.g. primary amine (-NH2), carboxyls (-COOH), sulfhydryls, or carbonyls (-CHO), or crosslinker reactive groups such as carbodiimide (e.g., EDC), NHS ester, imidoester, PFP ester, hydroxymethyl phosphine, maleimide, haloacetyl (bromo- or iodo-), pyridyldisulfide, vinyl sulfone, hydrazide, diazirine, aryl azide, or isocyantate.

Also envisaged by the present invention is that the linker molecule has a certain length so as to act as a spacer molecule, i.e. to provide a certain distance of the binding molecule and the magnetic particle surface. It is understood that the provision of such a distance may provide several other advantages:

> First, a certain distance may result in less unspecific binding of the magnetic particles to other molecules and to each other. Moreover, non-specific clustering of the magnetic particles which usually occur may be minimized.
>
> A second technical advantage of an enhanced end-to-end-distance between the binding molecule and magnetic particle surfaced is that the probability of binding a large magnetic particle to a surface such as a sensor surface as described herein can be significantly increased by attaching the target to the magnetic particle linker molecules having a certain length.

In preferred embodiments of the present invention a long and rigid linker molecule is used to overcome the binding difficulties in order to achieve a more effective binding. The effective binding is based on the observation that the number of possible orientations in which a magnetic particle can bind to the surface strongly increases if the attachment point is positioned further away from a magnetic particle. The use of long as well as rigid linker molecules for positioning a binding molecule away from the magnetic particle surface thus significantly enhances the kinetics of binding a magnetic particle to the surface. Thus, the resulting end-to-end-distance which is a function of the contour length and rigidity of a molecule contributes to the enhanced binding kinetics.

Linker molecules according to the present invention thus may have the function of a connector molecule and/or the function of a spacer. For this purpose, these polymeric molecules preferably have a certain length, strength, and/or rigidity in order to be able to act like polymeric fibers.

Also envisaged by the present invention is the use of polymers that assemble in fibers, e.g. by dimerization or higher order multimerization. Also envisaged is the use of "assisting molecules" which help to establish a higher order structure. One envisaged example is a single stranded nucleic acid molecule, which may assemble into a dimeric helix structure, and which in turn may assemble with an assisting molecule. Other envisaged examples for assisting molecules are proteins capable of binding nucleic acids such as to ssDNA, dsDNA or both (e.g. DNA binding proteins) or to ssRNA, dsRNA or both (e.g. RNA-binding proteins). The binding of such factors or proteins may preferably be guided by specific binding motifs recognized by the binding protein, which may be present in the nucleic acid molecule, e.g. the dsDNA or ssDNA or ssRNA. Once such binding factors are bound on the nucleic acid molecule, the rigidity of the linker may be enhanced and lead to linkers with increased average extension length. Such a linker modification may be carried out before or during an assay. In further specific embodiments of the present invention, assisting molecules such as RNA or DNA binding proteins may comprise protein-protein interaction domains, e.g. SH3, PDZ, 14-3-3, SH2 domains or any other suitable domain known to the person skilled in the art. Secondary binding factors, which comprise compatible recognition domains or interaction domains, may accordingly be provided allowing the assembly of complexes comprising nucleic acids bounds by assisting molecules, which in turn may be bound by secondary interacting molecules. In further embodiments, rigid linker structures may be provided in the form of polymeric polypeptides, e.g. based on the form and/or molecular identity of collagen, or collagen-like proteins such as Scl1, Scl2, SclA, or SclC. These molecules may further be combined with other linker molecules or functional groups as defined herein.

In further specific embodiments of the present invention, the polymeric linker molecules may be polymeric molecules capable of self-assembly under suitable conditions. Accordingly, the polymeric molecules may be provided in conjunction with magnetic particles leading to the assembly of the polymers on the surface of the magnetic particle. The self-assembly may be controlled by suitable parameters, e.g. the concentration of monomers or polymeric units necessary for the polymerization, the concentration of bridging factors for monomers or polymeric units, the pH of the reaction environment, the temperature, the presence of ions, the presence of assisting factors such as proteins, etc. The self-assembled polymers may, in further embodiments, be disintegrated upon the change of such a suitable condition, e.g. by the change of pH, the reduction or increase of the concentration of factors or monomers, the change of temperature etc.

It is also conceivable that the linker molecule comprises or consists of a charged structure as defined herein or be integrated in a steric surface structure as described herein.

In further specific embodiments of the present invention, the linker molecules may be linear, circular, or branched-like molecules, or a mixture thereof.

Suitable linker molecules within the context of the present invention may be, for example, nucleic acids, ribonucleic acids, peptides, polypeptides, proteins, carbohydrates, lipids, polyethylene glycol, polysaccharides, dendrimers, dendrons, nanotubes, or gold nanoparticles, or any suitable derivatives or combinations thereof.

Nucleic acid molecules as defined herein above may advantageously also be used "nucleic acid linker molecules". These linker molecules may comprise single and/or double stranded DNA molecules, or any type of derivative thereof as defined herein above. The DNA may, for example, be in the form of, e.g. A-DNA, B-DNA or Z-DNA or any mixture of these forms. The linker molecule may also be a PNA, CNA, HNA, LNA or ANA molecule, or any mixture or combination thereof, or any mixture or combination with other linker molecule as defined herein, e.g. with a any type of nucleic acid such as DNA or RNA. In specific embodiments, the linker molecule is a ribonucleic acid molecule, or any mixture of a ribonucleic acid with any of the other mentioned nucleic acid molecules or with any other spacer molecule as defined herein. The nucleic acid or ribonucleic acid molecules may be of different base compositions and/or lengths as defined herein above. The length of the linker molecule may be made dependent on the size or diameter of the magnetic particle, the presence, size or length of repulsive surface structure molecules as defined herein above, the envisaged overall specific net charge and/or steric repulsion of the magnetic particle, in particular since nucleic acid molecules if used as linkers may provide a specific net charge to the magnetic particle , any envisaged end-to-end-distance between binding molecule and particle surface, or any envisaged flexibility, rigidity or stability of the linker molecule. In certain embodiments of the present invention, the nucleic acid linker molecule may be a charged or un-charged molecule. The term "un-charged nucleic acid linker molecule" as used herein relates to a net charge of the molecule of about 0.

In a particular preferred embodiment of the present invention the linker molecule is or comprises a molecule selected from the group consisting of a double stranded or dsDNA, double stranded or dsRNA, PNA, a PNA-DNA duplex, and a RNA-DNA duplex.

One advantageous aspect, which is also envisaged by further preferred embodiments of the present invention, is that PNAs and PNA/DNA or PNA/DNA duplexes are not easily recognized by either nuclease or proteases making them resistant to enzymatic degradation.

Peptide molecules as defined herein above may advantageously also be used as "peptide linker molecules" in the context of the present invention. Such peptides may be of different amino acid compositions and/or lengths as defined herein above. The length of the peptide molecules linker molecule may be made dependent on the size or diameter of the magnetic particle, the presence, size or length of charged structure molecules as defined herein above, the envisaged overall specific net charge and/or steric repulsion of the magnetic particle, in particular since peptide molecules if used as linkers may provide a specific net charge and/or steric repulsion to the magnetic particle, any envisaged end-to-end-distance between binding molecule and particle surface, or any envisaged flexibility, rigidity or stability of the linker molecule. In certain embodiments of the present invention, the peptide linker molecule may be a charged or un-charged molecule. The term "un-charged peptide linker molecule" as used herein relates to a net charge of the molecule of about 0.

Protein or polypeptide molecules as defined herein above may advantageously also be used as "protein linker molecules" or "polypeptide linker molecules in the context of the present invention. Such proteins may be of different amino acid compositions and/or lengths as defined herein above. The length of the protein or polypeptide linker molecule may be made dependent on the size or diameter of the magnetic particle, the presence, size or length of charged structure molecules as defined herein above, the envisaged overall specific net charge and/or steric repulsion of the magnetic particle, in particular since protein or polypeptide molecules if used as linkers may provide a specific net charge and/or steric repulsion to the magnetic particle, any envisaged end-to-end-distance between binding molecule and particle surface, or any envisaged flexibility, rigidity or stability of the linker molecule. In certain embodiments of the present invention, the polypeptide or protein linker molecule may be a charged or un-charged molecule. The term "un-charged polypeptide or protein molecule" as used herein relates to a net charge of the molecule of about 0.

Carbohydrate molecules as defined herein above may advantageously also be used as "carbohydrate linker molecules" in the context of the present invention. Such carbohydrates as described herein above may be of different compositions and/or lengths as defined herein above. The length of the carbohydrate linker molecule may be made dependent on the size or diameter of the magnetic particle, the presence, size or length of charged structure molecules as defined herein above, the envisaged overall specific net charge and/or steric repulsion of the magnetic particle, in particular since carbohydrate molecule if used as linkers may provide a specific net charge and/or steric repulsion to the magnetic particle, any envisaged end-to-end-distance between binding molecule and particle surface, or any envisaged flexibility, rigidity or stability of the linker molecule. In certain embodiments of the present invention, the carbohydrate linker molecule may be a charged or un-charged molecule. The term "un-charged carbohydrate molecule" as used herein relates to a net charge of the molecule of about 0.

Lipid molecules as defined herein above may advantageously also be used as "lipid non-affine spacer molecules" in the context of the present invention. Such lipids may be of different compositions and/or lengths as defined herein above. The length of the lipid non-affine spacer molecule may be made dependent on the size or diameter of the magnetic particle, the presence, size or length of charged structure molecules as defined herein above, the envisaged overall specific net charge and/or steric repulsion of the magnetic particle, in particular since lipid molecules if used as linkers may provide a specific net charge and/or steric repulsion to the magnetic particle, any envisaged end-to-end-distance between binding molecule and particle surface, or any envisaged flexibility, rigidity or stability of the linker molecule. In certain embodiments of the present invention, the lipid spacer molecule may be a charged or un-charged molecule. The term "un-charged lipid molecule" as used herein relates to a net charge of the molecule of about 0.

Polyethylene glycol molecules as defined herein above may advantageously also be used as "polyethylene glycol linker molecules" in the context of the present invention. Such polyethylene glycols may be of different compositions and/or lengths as defined herein above. The length of the polyethylene glycol linker molecules may be made dependent on the size or diameter of the magnetic particle, the presence, size or length of charged structure molecules as defined herein above, the envisaged overall specific net charge and/or steric repulsion of the magnetic particle, in particular if polyethylene glycol molecule derivatives which carry electrical charges are used or any envisaged end-to-end-distance between binding molecule and particle surface, or any envisaged flexibility, rigidity or stability of the linker molecule. In certain embodiments of the present invention, the polyethylene glycol linker molecule, in particular a derivative thereof or modified version thereof, may be a charged or un-charged molecule. The term "un-charged polyethylene glycol molecule" as used herein relates to a net charge of the molecule of about 0.

"Dendrimers" or "dendrimetic linker molecules" may be any repeatedly branched, roughly spherical large molecules. Such dendrimers may be of different compositions and/or lengths, and/or degree of branching. The length may be made dependent on the envisaged overall specific net charge and/or steric repulsion, the envisaged end-to-end-distance between binding molecule and particle surface, the envisaged flexibility, rigidity or stability of the linker molecule, and/or the envisaged number of charged molecules per magnetic particle. Dendrimers as envisaged by the present invention may comprise low-molecular weight or high-molecular weight species. The dendrimers according to the present invention are preferably un-charged, i.e. the molecules show a net charge of 0.

Also envisaged by the present invention is the use of dendrimers according to the present invention as a part of a linker. For this purpose the dendrimers may, in certain embodiments, comprise functional groups on the molecular surface, e.g. hydrophilic groups, or alternatively have internal functionality. Dendrimers as envisaged for the purposes of the present invention may be dendrimers of 1st, 2nd, 3rd, 4th or a higher generation. Preferred dendrimers include, for example, newkome dendrimer or arbolol, and Polyamidoamine (PAMAM). It will be appreciated by one of skill in the art that dendrimers lead to structures that can be advantageously used to provide a voluminous and steric surface structure on the particle surface or flat surface wherein the linker molecule as described herein above is a part of such are structure or is embedded therein. Further variants to be used within the context of the present invention, as well as synthesis methods etc. would be known to the person skilled in the art or can be derived from suitable documents, such as "Dendrimers and other dendritic polymers"Frechet and Tomalia, J. Wiley.

"Dendrons" or" dendronic linker molecules" are understood as containing a single chemically addressable groups of dendrimers, i.e. a focal point of a dendrimer as defined herein above. The dendrons according to the present invention are preferably un-charged, i.e. the molecules show a net charge of 0. In particular embodiments of the present invention, dendrons may be used as a part of a linker as defined herein.

The term "nanotube" as used herein refers to carbon nanotubes, i.e. allotropes of carbon with a cylindrical nanostructure. Such nanotubes may be single walled nanotubes or multi walled nanotubes. The present invention also envisages linker molecules of the fullerene structural family of different types, forms and complexity, e.g. spherical, or ellipsoid buckyball forms, buckyball fullerene, or a combination of nanotubes with backbyballs etc. The nantotubes according to the present invention or other linker suitable linker molecules known to the person skilled in the art are preferably un-charged, i.e. the molecules show a net charge of 0.

In another preferred embodiment of the present invention the linker molecule may be longer than the repulsive surface structure, e.g. a charged nucleic acid molecule, peptide molecule, lipid molecule etc as defined herein. The term "longer" as used herein means that the average extension length of the linker molecule is larger than the average extension length of the surface structure. The difference in average extension length may be 5%, 10%, 15%, 20%, 30%, 50%, 75%, 100%, 200%, 300%, 500%, 1000% or more.

The term "average extension length" as used herein is defined as the root mean square end-to-end distance of the linker molecule $\sqrt{<R^2>}$, which can be described according to the worm-like chain model as:

$$<R^2> = 2\,Pl\,[1 - (P/l)\,(1 - e^{-l/P})],$$

wherein P is the persistence length of the polymer and l is the contour length of the linker.

The term "contour length" as used herein refers to the contour length of a polymer chain as the length at maximum physically possible extension.

Several biologically important polymers can be effectively modeled as worm-like chains, including double-stranded DNA and RNA, unstructured RNA and unstructured polypeptides. The Kratky-Porod-worm-like-chain-model is suitable for modeling of semi-flexible polymers in order to approximate end-to-end distances and persistence lengths. The skilled person is however aware of numerous other theoretical models, e.g. as described hereinafter, to approximate end-to-end distance and persistence lengths.

The term "persistence length, P" as used herein refers to a basic mechanical property quantifying the stiffness or rigidity of a polymer or a string and is defined as the length over which correlations in the direction of the tangent are lost. In chemistry, it can also be defined as the average sum of the projections of all bonds $j \geq i$ on bond i in an indefinitely long chain (Flory, Paul J. (1969), Statistical Mechanics of Chain Molecules, New York: Interscience Publishers). When the angle $\theta$ between a vector that is tangent to the polymer at position 0 (zero) and a tangent vector at a distance $L$ away from position 0, the expectation value of the cosine of the angle falls off exponentially with distance,

$$<\cos\theta> = e^{-(L/P)}$$

where P is the persistence length and the angled brackets denote the average over all starting positions. Flexible polymers such as PEG can be described using the Flory model (random walk).

The persistence length can be also defined using the bending stiffness $B_s$, the Young's modulus E and the section of the polymer chain as described in Mofrad, M.R.K, "Cytoskeletal mechanics: models and measurements", Cambridge Univ Press, 2006.

$$p_l = \frac{B_s}{k_B T}$$
$$B_s = EI$$

In the case of a rigid and uniform rod I can be expressed as:

$$I = \frac{\pi a^4}{4}$$

where *a* is the radius.

In polymer science, persistence length can be also defined as one half of the Kuhn length, i.e. the length of hypothetical segments that the chain can be considered as freely joined. The persistence length equals the average projection of the end-to-end-vector on the tangent to the chain contour at a chain end in the limit of infinite chain length.

As used herein "rigidity" or "rigid" also termed as "stiffness" define the property of a solid body to resist deformation. It is appreciated that rigidity of a linker molecule essentially contributes to the average extension length. "Flexible" as used herein thus refers to the property of a solid body, which can be easily deformed. In the context of the present invention, a flexible molecule, polymer, or linker thus has a low rigidity as defined herein which may result in a bending and fold up of the molecule.

In a specific embodiment of the present invention the rigidity of a linker molecule is determined via the root mean square end-to end distance of the linker $\sqrt{<R^2>}$ as defined above. In certain embodiments, the rigidity may accordingly be measured in terms of the linker's root means square end-to-end distance or average extension length in comparison to its contour length as defined herein. Thus, for example, a contour length of a linker molecule which is essentially identical to the average extension length of a linker molecule indicates a high rigidity of the linker molecule. On the other hand, a contour length of a linker molecule which is significantly larger than the average extension length of said linker indicates a low rigidity of the linker molecule.

It is envisaged by the present invention that the linker molecule accordingly has a certain length in order to provide an increased average extension length between the first binding molecule and the magnetic particle surface. It is conceivable that the effect provided by a concomitantly present repulsive structure, i.e. an increased repulsion, may also be achieved by, for instance, a high number of relatively short repulsive structures, e.g. a steric coating or charged molecules, being present on a magnetic particle as defined herein. The present invention thus envisages, in a preferred embodiment, a magnetic particle having long linker molecules and short repulsive surface structure molecules, e.g. a steric coating or charged molecules, in order to combine the advantageous features as described herein leading to an increased binding to the sensor surface. In further specific embodiments of the present invention the long linker molecules are combined with short charged molecules. In a further specific embodiment, the linker molecule may not have, at the same time, the function of a repulsive surface structure molecule. For example, the linker molecule may be an un-charged linker molecules as defined herein.

In certain embodiments of the present invention, the linker molecules may have an average extension length of at least about 60 nm, preferably up to about 500 nm, preferably of at least about 65 nm, 70 nm, 75 nm, 80 nm, 85 nm, 90 nm 95 nm or 100 nm, even more preferably of at least about 110 nm, 120 nm, 130 nm, 140 nm, 150 nm, 160 nm, 170 nm, 180 nm, 190 nm, or 200 nm, even more preferably of at least about 220 nm, 250 nm, 270 nm, or 300 nm, most preferably of at least 320 nm, 350 nm, 370 nm, or 400 nm. In further embodiments, the average extension length of the linker molecule may also be larger than 400 nm. The linker molecule may also have any other suitable average extension length in between the indicated values.

In another preferred embodiment of the present invention the linker molecule is or comprises a repulsive surface structure as defined herein. In order to obtain an increased binding the magnetic particles may integrate the advantageous features deriving from a long linker molecule and from the presence of repulsive surface structure e.g. by using linker molecules comprising a charged structure. This approach would thus have the advantage that only one type of molecule, which fulfils the requirements of a long linker and a repulsive surface structure, must be produced leading to a more homogenous and easy to produce particle. The present invention also envisages a combination of different types or forms of linker molecules and/or repulsive surface structures.

In yet another preferred embodiment of the present invention the repulsive surface structure is further functionalized with a first binding molecule capable of specifically binding to said target molecule. In a specific embodiment of the present invention, the repulsive surface structure, which is further functionalized with a first binding molecule, may increase or further increase the amount of first binding molecules on the magnetic particle. The first binding molecule is preferably be an antibody or a fragment thereof as defined herein above, an aptamer as defined herein above, or a complementary nucleic acid as defined herein above.

It is particularly preferred that the linker molecule and/or the repulsive surface structure is not cleavable by DNase and/or a restriction enzyme capable of cutting a double stranded nucleic acid molecule. Such a non-cleavability by DNase may be achieved by providing linker molecules or repulsive surface structures, which do not represent a substrate or recognition motif for a DNase enzyme. Typcially, the effect could be achieved by using DNA analogues such as PNA, CNA, HNA, LNA or ANA molecules as defined herein above.

A non-cleavability by a restriction enzyme capable of cutting a double stranded nucleic acid molecule may be achieved by providing linker molecules or repulsive surface structures which do not represent a substrate or recognition motif for restriction enzymes capable of cutting a double stranded nucleic acid molecule. Typically, the effect could be achieved by using DNA analogues such as PNA, CNA, HNA, LNA or ANA molecules as defined herein above, or by using DNA molecules which are single stranded, or by using DNA molecules which do not contain a specific sequence motif necessary for the binding and/or activity of a restriction enzyme. Further details would be known to the person skilled in the art, or can be derived from suitable literature sources.

In further specific embodiments of the present invention the linker molecule as defined herein and/or the repulsive surface structure as defined herein is not cleavable by RNase. Such a non-cleavability by RNase may be achieved by providing linker molecules or repulsive surface structures, which do not represent a substrate or recognition motif for an RNase enzyme.

In a further preferred embodiment of the present invention the nucleic acid molecule such as dsDNA, a PNA molecule, a PNA-DNA duplex, and an RNA-DNA duplex has a length of about 50 to 1000 nucleotides. The nucleic acid molecule such as dsDNA, a PNA molecule, a PNA-DNA duplex, and an RNA-DNA duplex may, for example, have length of about 50, 60, 70, 80, 90, 100, 110, 120, 130, 140, 150, 160, 170, 180, 190, 200, 220, 250, 270, 300, 320, 350, 370, 400, 420, 450, 470, 500, 520, 550, 570, 600, 620, 650, 670, 700, 720, 750, 770, 800, 820, 850, 870, 900, 920, 950, 970 or 1000 nucleotides, or any length in between the indicated values. In further specific embodiments, the nucleic acid molecule such as dsDNA, PNA, PNA-DNA duplex, or RNA-DNA duplex may also be shorter or longer than the indicated values.

In a particularly preferred embodiment of the present invention the nucleic acid molecule such as dsDNA, a PNA molecule, a PNA-DNA duplex, and an RNA-DNA duplex has a length of about 100 nucleotides.

In a particularly preferred embodiment of the present invention the nucleic acid molecule is selected from the group consisting of dsDNA, a PNA molecule, a PNA-DNA duplex, and an RNA-DNA duplex.

It is understood that the objective as set out above is also solved by a second aspect of the present invention which describes a device for detecting a target molecule within a sample comprising a mixture of at least two types of magnetic particles, wherein one type is functionalized with a first binding molecule capable of specifically binding to said target molecule, wherein said first binding molecule is attached to the particle, and a second type is functionalized with a repulsive surface structure, which is directly attached to the surface of said particle, wherein said repulsive surface structure covers the surface of the magnetic particle so as to result in a specific net charge and/or steric repulsion of the magnetic particle, and a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface.

The definitions and embodiments set out herein above for the first aspect of the invention also apply to the second aspect of the present invention. The second aspect of the present invention is based on the same principle, namely on the generation of an electrostatic and/or steric pushing effect on said magnetic particles towards said sensor surface. However, this effect is achieved by the provision of a mixture of at least two types of magnetic particles.

The term "mixture of at least two types of magnetic particles" as used herein means that in addition to a magnetic particle functionalized with a first binding molecule, a second or further type of magnetic particles which is primarily functionalized by a repulsive surface structure as defined herein (repulsive magnetic particle) may be employed in a device as described herein above. In specific embodiments, the repulsive magnetic particles of this second or further type are not functionalized by a first binding molecule. Such magnetic particles may accordingly only comprise a repulsive surface structure without being functionalized, especially when used in combination with a functionalized magnetic particles.

The present invention envisages, in a specific embodiment, the use of only one second type of repulsive magnetic particle together with only one first type of magnetic particle functionalized with a first binding molecule. Also envisaged is the use of more than one type of repulsive magnetic particles together with only one first type of magnetic particles functionalized with a first binding molecule. For example, the repulsive magnetic particles having different repulsive surface structures, or comprising different molecules such as different charged molecules or steric coatings as defined herein above may be used. Furthermore, the repulsive magnetic particles may, for example, differ in size or diameter, magnetic properties etc. Further envisaged is the use of one or more than one type of repulsive magnetic particle together with only one or more types of magnetic particles functionalized with a first binding molecule. These one or more types of magnetic particles functionalized with a first binding molecule may, for example, differ in the first binding molecule, size or diameter, magnetic properties etc.

Such an approach may have the major advantage that the repulsive magnetic particles are clearly separated from their function as capturing particles. In other words, the functionalities leading to the improved binding properties are now separated and distributed on two or more types of particles. One advantage may thus be that the molecule of the repulsive surface structure is unspecific regarding the target molecule and can be used more versatile. The repulsive magnetic particles may for instance be added to non-repulsive magnetic particles being specific for a target molecule of interest. It is accordingly envisaged by the present invention that the repulsive magnetic particles of the resulting mixture of magnetic particles may act to form an electrostatic pushing effect so that magnetic particles functionalized with the binding molecule are pushed towards the sensor surface.

In specific embodiments of the present invention, the first type of magnetic particles functionalized with a first binding molecule capable of specifically binding to said target molecule may additionally comprise a repulsive surface structure such as a charged molecule or a steric coating as defined herein above.

In further specific embodiments of the present invention, the first type of magnetic particles functionalized with a first binding molecule capable of specifically binding to said target molecule may additionally comprise charged molecules as defined herein above.

The ratio of first type to second type magnetic particles, e.g. of charged and non-charged magnetic particles within a mixture of at least two types of magnetic particles may be between 10:90 and 90:10. For example, a ratio of first type : second type magnetic particles, e.g. of charged and non-charged magnetic particles, may be a ratio of 10 : 90, 20 : 80, 30 : 70, 40 : 60, 50 : 50, 60 : 40, 70: 30, 80 : 20, or 90: 10. Also envisaged is any other ratio in between the indicated values.

In another preferred embodiment of the present invention the linker molecule as defined herein further comprises at least one short flexible spacer. It is accordingly envisaged by the present invention that linker structures as defined herein, preferably long and rigid linkers as define herein, carry at one or a both ends a short flexible spacer. It is appreciated that a certain flexibility due to the presence of such flexible spacer is added to the rigid linker molecule. The flexible spacer structures may thus serve as a flexible joint which contributes to the mobility of the linker molecule while retaining its overall rigidity. The number of possible orientations of the linker molecule may be facilitated by such a flexible spacer. The flexible spacer may be any suitable flexible molecule, e.g. a polymeric molecule, peptide, chemical group etc.

In a particularly preferred embodiment the short flexible spacer comprises a single stranded nucleic acid, e.g. a single stranded DNA or RNA molecule as defined herein, or any other single stranded nucleic acid form as defined herein or as known to the person skilled in the art.

In a further aspect the present invention relates to a method of detecting the presence or amount of a target molecule within a sample comprising the steps of contacting the sample and a first binding molecule attached to a magnetic particle in a device as described herein and contacting the sample with a second binding molecule capable of attaching to a sensor surface, wherein the first binding molecule and/or the second binding molecule are capable of specifically binding to said target molecule, and optionally a target analog molecule attached to the sensor surface; wherein the target molecule is capable of interfering with the binding of the first binding molecule to the target analog molecule, optionally adding magnetic particles comprising a repulsive surface structure as defined herein and detecting the number of particles bound to the sensor surface, wherein a magnetic force is applied to bring the particle into close proximity with the sensor surface; and wherein the number of bound particles is directly or inversely related to the amount of target molecules present in the sample.Envisaged by the present invention are assay formats, which can be either typical competitive or non competitive assays for detection of the presence and/or amount of a target molecule. It will be appreciated by the person skilled in the art that the device as described herein above as well as the method according to the present invention are suitable to carry out such an analysis.

The term "capable of attaching to the sensor surface" means that the second binding molecule is not necessarily constantly bound to the sensor surface attached to it in a predetermined manner, i.e. not necessarily attached to the surface already at the beginning of the assay. It is understood that the attachment of the second binding molecule to the surface either directly or via a linker molecule as defined herein above may occur at any time point during the assay. It will be appreciated by one of skill in the art that a selective time point of attachment opens up a broad range of possibilities to conduct an assay. Envisaged by the present invention is thus a second binding molecule-linker complex that may act as a separate module independent of its binding to the surface. It will further be appreciated by the skilled person that a second binding molecule-linker complex that is not constantly fixed or attached at the sensor surface may have several advantages. One envisaged advantage is that the unattached second binding molecule-linker complex can diffuse freely and fast, thus enhancing the binding to the capture complex via binding to the target molecule. The term "capture complex" describes a state where the first binding molecule or capture molecule that is directly or indirectly attached to a first magnetic particle having captured the target molecule. In this example, the binding of the second binding molecule/ linker module to the target molecule that was captured by the first binding molecule may occur timely independent, i.e. occur prior to its attachment to the surface.

In particular preferred embodiments the attachment of said second binding molecule to the sensor surface may occur prior or after the binding of the second binding molecule to the target molecule. In specific embodiments of the present invention, the second binding molecule is allowed to attach to the surface, preferably via a linker molecule as defined herein, in a first step of the assay or prior to the begin of the assay, which is the time point when the sample as defined herein above is added. In such a case, the capture complex is formed first and binds in a subsequent step to a preformed surface structure comprising the second binding molecule attached to the surface.

In further preferred embodiments of the present invention, the second binding molecule-linker module binds to the capture complex by virtue of its specific binding to the target molecule first and the entire capture - second binding molecule - linker complex is subsequently guided to the sensor surface so as to link the capture complex to the surface. The distance may be generated by a long linker attached to the second antibody, while the first binding molecule is attached to the surface of the first particle directly. In other words, the second binding molecule having attached a linker generates the effect leading to enhanced binding kinetics.

In the envisaged non-competitive assay format both the first and second binding molecule are capable of specifically binding to the target molecule. It is conceivable that in a first step, the first binding molecule present on a first magnetic particle may recognize and bind the target. In a second step the first magnetic particle having bound the target molecule may be directed by diffusion or magnetic actuation to a second binding molecule present the sensor surface. Binding to the sensor surface is mediated by the binding of the second binding molecule to the target. Also envisaged are one or more washing steps, where unspecifically bound magnetic particles are removed from the surface. In specific embodiments of the present invention such washing steps occur via pulsed magnetic actuation. The number of particles is subsequently bound to the sensor surface is subsequently determined. The number of bound particles correspond directly to the number of target molecules present in the sample.

Also envisaged are assay formats based on a competitive assay, which typically require the presence of a competitor or a target analog. The term "target analog molecule" as used herein refers to any molecule, which competes with the target molecule for the binding to the first binding molecule as defined herein. In such a case the target molecule may interfere with the binding of the first binding molecule to the target analog molecule. Envisaged by the present invention are competition or inhibition assay formats, where the first binding protein may bind a target analog molecule attached on the surface of the flat surface or a magnetic particle as defined herein above. This binding can be prevented if a target molecule as described herein is present in the sample and if this target molecule binds to the antibody first. For example, a small drug molecule can be detected using the envisaged method if an analog of the drug molecule is e.g. immobilized to the flat sensor surface. If no drug molecule is present in the sample, all particles with the binding molecule recognizing the target molecule may bind to the target analog molecule on the surface. However, in the presence of drug (target) molecules, which interfere with this binding, the anti-drug binding molecules cannot bind or bind less to the target analog on the surface. In such a case the amount of particles on the surface is inversely related to the amount of target molecules.

Envisaged by the present invention is that the magnetic particles can be actuated by applying a magnetic field such that the analytical procedure can be accelerated. It is also envisaged by the present invention that the use of a magnetic field may be reduce the background signal due to removal of unspecifically bound particles. An exemplary optomagnetic system suitable for the method of detection according to the present invention is illustrated in Fig. 1.

Particularly preferred are sensing devices based on an optical detection of magnetic particles. Corresponding details may be derived from the exemplary device illustrated in Fig. 1, which comprises a light source and a light detection system. The optical methods used for detection typically measure the a change in light signal that means a difference in light reflected from the magnetic particles and which can be detected by optical means. For instance, such methods may include techniques such as the detection of scattered light or detection based on total internal reflection (TIR) or frustrated total internal reflection (FTIR). Preferably, the change in light signal refers to only those magnetic particles being bound by virtue of the binding of the second binding molecule to the sensor surface. Details would be known to the person skilled in the art, or can be derived from suitable references, such as Bruls et al., Lab Chip, 2009, 9. 2504-3510.

As used herein the term "total internal reflection" describes a condition present in certain materials when light enters one material from another material with a higher refractive index at an angle of incidence greater than a specific angle. The specific angel at which this occurs depends on the refractive indices of both material, also referred to as critical angel and can be calculated mathematically (Snell's law, law of refraction). In absence of magnetic particles no refraction occurs and the light beam from the light source is totally reflected. If a magnetic particle is close to the surface or is in contact with the surface the light rays are said to be frustrated by the particle and reflection at that point is no longer total.

The signal, which may be defined as the decrease of the totally internal reflected signal can be calculated. The signal is more or less linearly dependent on the concentration of particles on the surface (surface density ñ). The signal can be expressed as:

$$S = \beta\, \tilde{n}$$

wherein S is the measured signal change in % and $\beta$ is a conversion factor from surface density to signal change.

In a preferred embodiment of the present invention detection of bound particles occurs via frustrated total internal reflection (FTIR) or via measurement of scattered light from said bound particles near the surface.

In another aspect the present invention relates to the use of a magnetic particle as defined herein for detecting a target molecule within a sample, e.g. a sample as defined herein above.

In a particularly preferred embodiment of the present invention the target molecule as mentioned in the context of the device, method or use as described herein above is cardiac troponin I (cTnI), NT-proBNP or parathyroid hormone. The present invention also aims at the detection of further target molecules or classes or target molecules. Particularly preferred is cardiac troponin I (cTnI).

EXAMPLES

*Example 1 — Effect of negatively charged DNA molecules on zeta potential*

The effect of negatively charged DNA molecules was analyzed. To this end highly negatively charged DNA molecules were bound to the particle. The charge on the particles was measured as the zeta potential. The charge increases when the amount of highly negatively charged DNA molecules bound to the particle is increased. Furthermore, higher charges are measured for particles carrying longer DNA molecules (bearing more charges).

Zeta potential values for 500-nm Ademtech particles, functionalised with streptavidin molecules and bound to different amounts of biotin-functionalised dsDNA of varying lengths were measured in 10mM phosphate buffer, at a pH 7.4 (see Fig. 4).

*Example 2 — Effect of increasing number of 97 bp dsDNA*

In order to determine effects of increasing number of 97 bp dsDNA FTIR signal amplitudes for assays performed with varying amounts of biotin-tagged 97 bp dsDNA attached to 500 nm Ademtech superparamagnetic particles, functionalised with streptavidin proteins were determined. The more DNA molecules per particle, the larger the surface contact, as can be seen in Fig. 6.

*Example 3 — Signal change at the end of an FTIR assay*

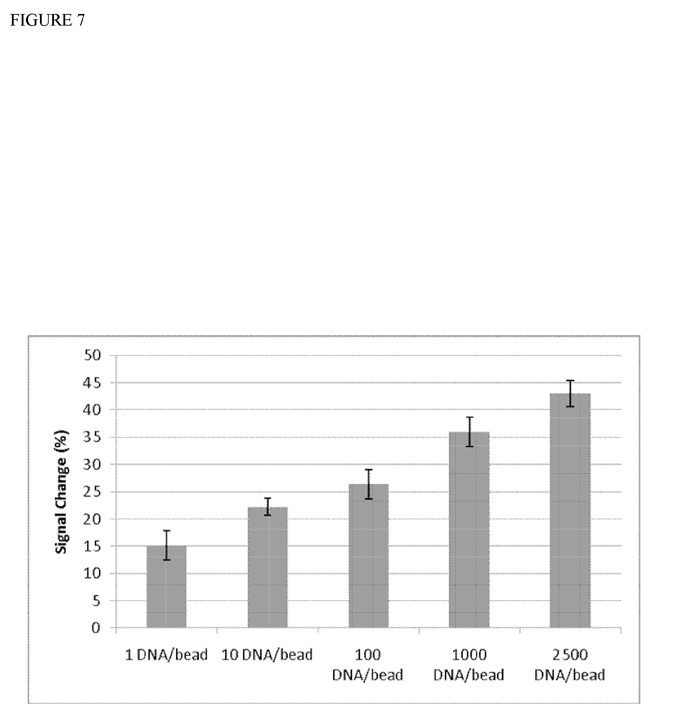

In a further experiment the signal change at the end of an FTIR assay. First, a single 97bp dsDNA fragment carrying a biotin at one end of the molecule and a Texas-Red molecule at the other end of the DNA was bound to the streptavidin particle. Subsequently, similar 97 bp DNA molecules, but lacking the Texas-Red molecule, were added per particle to the indicated amount. All particles were tested in an FTIR assay with a printed spot of anti-Texas Red antibodies (see Fig. 7).

*Example 4*— *Effect of negatively charged DNA molecules on* hydrodynamic size

The effect of negatively charged DNA molecules on the hydrodynamic size of particles was analyzed. To this end highly negatively charged DNA molecules were bound to the particle. The hydrodynamic size or hydrodynamic radius of the particles was measured. The hydrodynamic size or hydrodynamic radius increases when the amount of DNA molecules bound to the particle is increased.

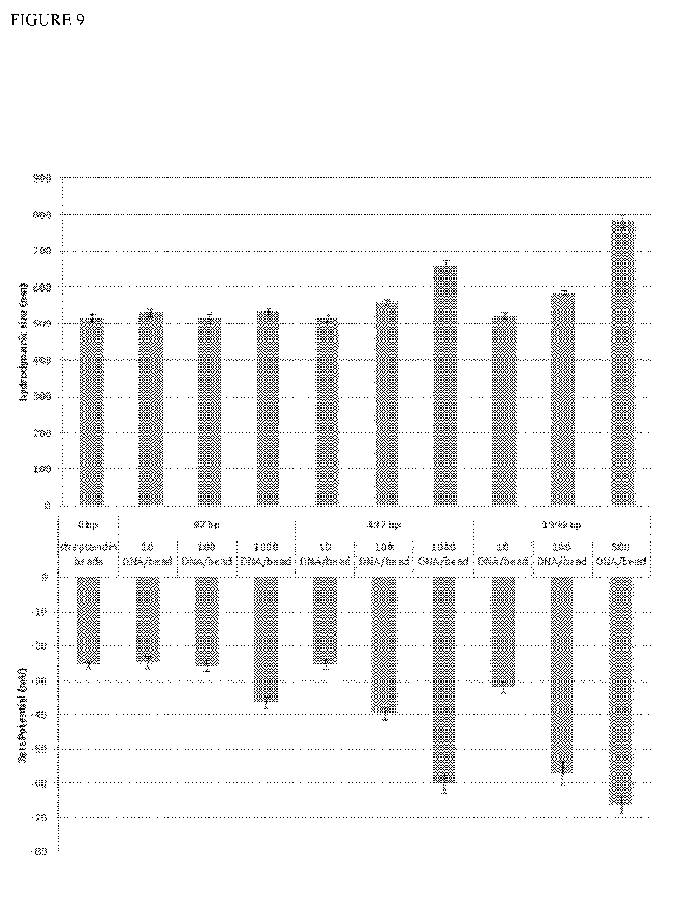

Hydrodynamic sizes and corresponding zeta potential values for 500-nm Ademtech particles, functionalised with streptavidin molecules and bound to different amounts of biotin-functionalised dsDNA of varying lengths were measured in 10mM phosphate buffer, at a pH 7.4 (see Fig. 9).

**Claims**

1. A device for detecting a target molecule within a sample comprising

    (a) a sample container for the measurement of the target molecule within a sample,
    (b) a magnetic particle, wherein said particle is functionalized with

    (i) a first binding molecule capable of specifically binding to said target molecule, wherein said first binding molecule is attached to the particle, and
    (ii) a repulsive surface structure, which is directly attached to the surface of said particle, wherein said repulsive surface structure covers the surface of the magnetic particle so as to result in a specific net charge and/or steric repulsion of the magnetic particle, and

    (c) a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface.

2. A device for detecting a target molecule within a sample comprising

    (a) a sample container for the measurement of the target molecule within a sample,
    (b) a mixture of at least two types of magnetic particles, wherein

    (i) one type is functionalized with a first binding molecule capable of specifically binding to said target molecule, wherein said first binding molecule is attached to the particle, and
    (ii) a second type is functionalized with a repulsive surface structure, which is directly attached to the surface of said particle, wherein said repulsive surface structure covers the surface of the magnetic particle so as to result in a specific net charge and/or steric repulsion of the magnetic particle, and

    (c) a sensor surface comprising a second binding molecule, wherein said magnetic particles are capable of binding said second binding molecule of the sensor surface directly or indirectly; wherein the number of bound particles is directly or inversely related to the amount of target molecules present in the sample; and wherein said repulsive surface structure conveys an electrostatic and/or steric pushing effect on said magnetic particles towards said sensor surface.

3. The device of claim 1 or 2, wherein said first binding molecule is attached to the particle via a linker molecule, wherein preferably said linker molecule is longer than the repulsive surface structure.

4. The device of claim 3, wherein said linker molecule is or comprises said repulsive surface structure.

5. The device of any one of claims 1 to 4, wherein said repulsive surface structure is further functionalized with a first binding molecule capable of specifically binding to said target molecule.

6. The device of any one of claims 1 to 5, wherein said pushing effect is determined by an increase of the surface interaction by a least 1%.

7. The device of any one of claims 1 to 6, wherein said repulsive surface structure is a charged structure or a steric coating.

8. The device of claim 7, wherein (i) said charged structure conveys a zeta potential to said magnetic particle of at least about 25 mV and/or (ii) said steric coating conveys a hydrodynamic radius of said magnetic particle of at least about 105% of the radius of said magnetic particle.

9. The device of any one of claims 1 to 8, wherein said first binding molecule is an antibody or a fragment thereof, an aptamer or a complementary nucleic acid.

**10.** The device of any one of claims 1 to 9, wherein said charged structure is or comprises a molecule selected from the group consisting of a nucleic acid molecule, such as dsDNA, a PNA molecule, a PNA-DNA duplex, and an RNA-DNA duplex, a hydro-gel, and a polymer.

**11.** The device of any one of claims 3 to 9, wherein said linker molecule and/or repulsive surface structure is not cleavable by DNase and/or a restriction enzyme capable of cutting a double stranded nucleic acid molecule.

**12.** The device of claim 10 or 11, wherein said nucleic acid molecule, preferably dsDNA, a PNA molecule, a PNA-DNA duplex, and an RNA-DNA duplex, has a length of about 50 to 1000 nucleotides, preferably of about 100 nucleotides.

**13.** Method of detecting the presence or amount of a target molecule within a sample comprising the steps of

(a) contacting the sample and a first binding molecule attached to a particle in a device of any one of claims 1 to 12, and
(b) contacting the sample with

i) a second binding molecule capable of attaching to a sensor surface, wherein the first binding molecule and/or the second binding molecule is capable of specifically binding to said target molecule, and optionally
ii) a target analog molecule attached to the sensor surface; wherein the target molecule is capable of interfering with the binding of the first binding molecule to the target analog molecule; optionally adding magnetic particles comprising a repulsive surface structure according to claim 1(b) (ii) or 2(b) (ii) and

(c) detecting the number of particles bound to the sensor surface,
wherein a magnetic force is applied to bring the particle into close proximity with the sensor surface; and wherein the number of bound particles is directly or inversely related to the amount of target molecules present in the sample.

**14.** Use of a magnetic particle as defined in any one of claims 1 to 12 for detecting a target molecule within a sample.

**15.** The device of any one of claims 1 to 12, the method claim 13 or the use of claim 14, wherein said target molecule is cardiac troponin I (cTnI), NT-proBNP or parathyroid hormone.

FIGURE 1

FIGURE 2

FIGURE 3

FIGURE 4

FIGURE 5

FIGURE 6

FIGURE 7

FIGURE 8

FIGURE 9

Europäisches
Patentamt

European
Patent Office

Office européen
des brevets

## EUROPEAN SEARCH REPORT

Application Number

EP 11 19 1453

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/148863 A1 (XU XIAOHONG NANCY [US] ET AL) 11 June 2009 (2009-06-11) | 14 | INV. G01N33/543 |
| Y | * claims *<br>* paragraph [0011] - paragraph [0012] *<br>* paragraphs [0041], [0144]; figure 1 *<br>* paragraph [0178] - paragraph [0179]; figure 10 *<br>----- | 1-15 | |
| Y | JEROEN NIEUWENHUIS: "Magnotech: Reliable and Fast Magnetic Point-of-Care Biosensor Technology",<br>INTERNET CITATION,<br>16 April 2009 (2009-04-16), pages 1-10, XP002610327,<br>Retrieved from the Internet:<br>URL:http://www.aacc.org/events/meeting_proceeding/2009/Documents/NieuwenhuisOakRidge.pdf<br>[retrieved on 2010-11-18]<br>* the whole document *<br>----- | 1-15 | |
| Y,D | BRULS D M ET AL: "Rapid integrated biosensor for multiplexed immunoassays based on actuated magnetic nanoparticles",<br>LAB ON A CHIP, ROYAL SOCIETY OF CHEMISTRY, CAMBRIDGE, GB,<br>vol. 9, no. 24,<br>1 January 2009 (2009-01-01), pages 3504-3510, XP002583698,<br>ISSN: 1473-0197, DOI: 10.1039/B913960E<br>[retrieved on 2009-10-15]<br>* page 3505; figure 1 *<br>* page 3509, left-hand column, paragraph 3 - right-hand column, paragraph 3 *<br>----- | 1-15 | TECHNICAL FIELDS SEARCHED (IPC)<br><br>G01N |

The present search report has been drawn up for all claims

| Place of search | Date of completion of the search | Examiner |
|---|---|---|
| The Hague | 22 February 2012 | Routledge, Brian |

EPO FORM 1503 03.82 (P04C01)

## ANNEX TO THE EUROPEAN SEARCH REPORT
## ON EUROPEAN PATENT APPLICATION NO.

EP 11 19 1453

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2012

| Patent document cited in search report | Publication date | Patent family member(s) | Publication date |
|---|---|---|---|
| US 2009148863 A1 | 11-06-2009 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUCHINI et al.** *Nano Lett.,* 2008, vol. 8 (1), 350-361
- **BRULS et al.** *Lab Chip,* 2009, vol. 9, 2504-3510
- **BRULS et al.** *Lab Chip,* 2000, vol. 9, 2504-3510
- **RANZONI et al.** *Nano Lett.,* 2011, vol. 11, 2017-2022
- **BRULS et al.** *Lab Chip,* 2009, vol. 9, 3504-3510
- **NIELSEN PE ; EGHOLM M.** An Introduction to Peptide Nucleic Acid. *Curr. Issues Mol. Biol.,* 1999, vol. 1 (2), 89-104
- **HERMANSON.** Bioconjugate techniques. Academic Press, Elsevier
- **FRECHET ; TOMALIA.** Dendrimers and other dendritic polymers. J. Wiley
- **DEWAR, M.J.S.** The Molecular Orbital Theory of Organic Chemistry. McGraw-Hill, and Inc, 1969
- **STEWART, R.** The Proton: Applications to Organic Chemistry. Academic Press, Inc, 1985, 72
- Protein Identification and Analysis Tools on the ExPASy Server. **GASTEIGER E. et al.** The Proteomics Protocols Handbook. Humana Press, 2005
- **FLORY, PAUL J.** *Statistical Mechanics of Chain Molecules,* 1969
- **MOFRAD, M.R.K.** Cytoskeletal mechanics: models and measurements. Cambridge Univ Press, 2006